# EUROPEAN PATENT APPLICATION

(11) **EP 4 644 384 A1**
(43) Date of publication of application: **05.11.2025**
(21) Application number: 23914516.2
(22) Date of filing: 21.12.2023
(51) Int. Cl.: C07D 401/14, A61K 31/4545, A61P 35/00

(54) **CYP11A1 INHIBITOR COMPOUND, AND PREPARATION METHOD THEREFOR AND USE THEREOF**

(30) Priority: 06.01.2023 CN 202310019758; 17.01.2023 CN 202310060807; 07.06.2023 CN 202310670257
(71) Applicant: Shanghai Lichun Biotech Co., Ltd., Shanghai 200030 (CN)
(72) Inventor: YU, Shanghai, Shanghai 200030 (CN)
(74) Representative: Engelhard, Markus
(86) International application number: PCT/CN2023/140517
(87) International publication number: WO 2024/146383

(57) **Abstract**

Disclosed in the present invention are a compound as represented by general formula (I), and a stereoisomer, a tautomer, a deuterated derivative or a pharmaceutically acceptable salt thereof. Compared with the prior art, the compound of the present invention has a new skeleton. The compound of the present invention unexpectedly shows a good inhibitory effect on CYP11A1 and blocks the biosynthesis of downstream steroids. Clinically, the compound can be used for treating steroid receptor-dependent diseases.

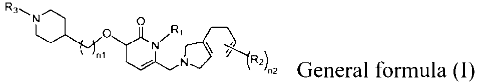

## Description

The present application claims the benefit of priority of a China patent with application no. 202310060807.3, with the title of disclosure "CYP11A1 INHIBITOR COMPOUND, AND PREPARATION METHOD THEREFOR AND USE THEREOF", filed with China National Intellectual Property Administration on January 17, 2023, the benefit of priority of a China patent with application no. 202310019758.9, with the title of disclosure "CYP11A1 INHIBITOR COMPOUND, AND PREPARATION METHOD THEREFOR AND USE THEREOF", filed with China National Intellectual Property Administration on January 06, 2023, and the benefit of priority of a China patent with application no. 202310670257.7, with the title of disclosure "CYP11A1 INHIBITOR COMPOUND, AND PREPARATION METHOD THEREFOR AND USE THEREOF", filed with China National Intellectual Property Administration on June 07, 2023, which are incorporated herein in their entireties by reference.

### TECHNICAL FIELD

The present disclosure relates to the field of drug synthesis, in particular to a CYP11A1 (cytochrome P450 monooxygenase 11A1) inhibitor compound, and a preparation method for and use thereof, and a pharmaceutical composition comprising such a compound, the compound can be used for treating a steroid hormone-dependent disease.

### BACKGROUND ART

Prostate cancer is one of the common malignant tumors in the genitourinary system of elderly males. The incidence and mortality thereof rank second and fifth respectively in the incidence and death spectra of male malignant tumors in the world, rank first and third respectively in males in European and American countries, and rank sixth and seventh respectively in males in China. In recent years, with the intensified aging of the population in China and other reasons, the incidence and mortality of prostate cancer have shown a significant upward trend, and the disease burden has been increasingly severe. GLOBOCAN2020 data have shown that there were about 115,000 new cases of prostate cancer in China in 2020, accounting for 4.7% of all male malignant tumors, and in 2019, new cases of prostate cancer in the United States accounted for 20% of male patients with new cancer that year.

For patients with stage I-III prostate cancers, existing standard treatments include surgery, radiotherapy, etc. The therapeutic effect of early stage prostate cancer (stage I/II) is relatively good, with a 5-year disease-free progression period of up to 90%. However, the cure rate of advanced prostate cancer is very low. At present, the 5-year survival rate of advanced prostate cancer is only 30%.

The binding of steroid hormones to their cognate receptors regulates the growth of most prostate and breast cancers. For patients with stage IV or high-risk prostate cancer, androgen deprivation by surgery or chemical castration can effectively control the progress of the disease. First-generation antiandrogen therapies, such as flutamide and bicalutamide, are used for treating prostate cancer at this stage.

Most prostate patients eventually develop into castration-resistant prostate cancer (CRPC). Castration reduces the level of testosterone in plasma, but the disease still progresses. It is characterized by the high expression of androgen receptor AR, and a trace amount of androgen in the body can still activate the androgen receptor AR signaling, thus leading to continuous activation of the AR pathway.

At present, treatment means for CRPC are limited. Abiraterone and enzalutamide are new therapies developed for the continuous activation of androgen receptor in CRPC, which can treat advanced CRPC by further inhibiting androgen synthesis or blocking the binding of a trace amount of androgen to the receptor in the body.

However, a considerable fraction of prostate patients are insensitive to abiraterone or enzalutamide. In addition, most patients with initial response will also develop new resistance after 1-2 years of using abiraterone or enzalutamide. However, most abiraterone- or enzalutamide-resistant tumors still have highly expressed AR in a continuously activated state.

Adrenal and prostate cancers themselves can synthesize and transform pregnenolone, progesterone, dehydroepiandrosterone, and derivatives thereof into more active androgen that binds and activates AR. The increase of pregnenolone in patients treated with abiraterone is considered to be the main mechanism of driving resistance, especially in tumors with point mutations in androgen receptor. All steroid hormones in the body begin with the transformation of a single precursor - cholesterol - into pregnenolone. This step of reaction is catalyzed by cytochrome P450scc (also called cholesterol side-chain cleavage enzyme, or cytochrome P450 monooxygenase 11A1, CYP11A1) and is the first step of continuous monooxygenation reaction, as a rate-limiting step, to catalyze steroid production, by which C-C in 20R,22R-dihydroxycholesterol (20R,22R-DiOHCH) is cleaved and the cholesterol is converted into pregnenolone. Studies have confirmed that inhibition of CYP11A1 can quickly reduce the level of steroid hormones in the blood below the detection limit. Supplementing the body with glucocorticoids and mineralocorticoids at the same time can effectively prevent the normal physiological function of the body from being affected by steroid hormone deficiency.

CYP11A1 belongs to the steroid CYP gene family. CYP11A1 is over-expressed in several types of cancer and is related to resistance. Inhibition of CYP11A1 enzyme can result in inhibited synthesis of all steroid hormones, including estrogen and progesterone, which promote the progress of breast cancer; androgen, which promotes the progress of prostate cancer; etc., thus fully inhibiting the progress of steroid hormone-dependent tumors. It is a promising therapeutic target.

### SUMMARY

According to one aspect of the present disclosure, an object of the present disclosure is to provide a compound represented by general formula (I), or a stereoisomer, tautomer, deuterated derivative or pharmaceutically acceptable salt thereof, 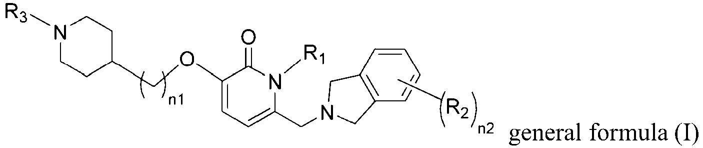
R₁ is selected from a hydrogen atom, C₁₋₆ alkyl, C₃₋₆ cycloalkyl, C₆₋₁₄ aryl, 4- to 8-membered heterocyclyl containing 1 to 3 heteroatoms selected from N, O, and S, 5- to 8-membered heteroaryl containing 1 to 3 heteroatoms selected from N, O, and S, wherein the C₁₋₆ alkyl, C₃₋₆ cycloalkyl, C₆₋₁₄ aryl, 4- to 8-membered heterocyclyl, and 5- to 8-membered heteroaryl are optionally substituted with one or more Rₐ substituents,
each Rₐ is the same as or different from each other and is independently selected from hydrogen, deuterium, tritium, halogen, amino, hydroxyl, cyano, C₁₋₆ alkoxy, C₁₋₆ alkyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy;
R₂ is selected from a hydrogen atom, a halogen atom, C₁₋₆ alkyl, C₂₋₆ alkenyl, C₂₋₆ alkynyl, C₁₋₆ alkoxy, C₃₋₆ cycloalkyl, C₆₋₁₄ aryl, 4- to 8-membered heterocyclyl containing 1 to 3 heteroatoms selected from N, O, and S, 5- to 8-membered heteroaryl containing 1 to 3 heteroatoms selected from N, O, and S, wherein the C₁₋₆ alkyl, C₂₋₆ alkenyl, C₂₋₆ alkynyl, C₁₋₆ alkoxy, C₃₋₆ cycloalkyl, C₆₋₁₄ aryl, 4- to 8-membered heterocyclyl, and 5- to 8-membered heteroaryl are optionally substituted with one or more R_{b} substituents,
each R_{b} is the same as or different from each other and is independently selected from hydrogen, deuterium, tritium, halogen, amino, hydroxyl, cyano, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy;
R₃ is selected from C₁₋₇ alkylcarbonyl, C₂₋₇ alkenylcarbonyl, C₂₋₇ alkynylcarbonyl, C₁₋₇ alkoxycarbonyl, C₃₋₇ cycloalkylcarbonyl, sulfonic acid group, aminosulfonyl, 3- to 8-membered heterocycloalkylcarbonyl containing 1 to 3 heteroatoms selected from N, O, and S, NR_{c}R_{d} carbonyl, C₁₋₇ alkyl S(O)₂-, C₂₋₇ alkenyl S(O)₂-, C₂₋₇ alkynyl S(O)₂-, C₁₋₇ alkoxy S(O)₂-, C₃₋₇ cycloalkyl S(O)₂-, 3- to 8-membered heterocycloalkyl S(O)₂- containing 1 to 3 heteroatoms selected from N, O, and S, NR_{c}R_{d}S(O)₂-, wherein the C₁₋₇ alkylcarbonyl, C₂₋₇ alkenylcarbonyl, C₂₋₇ alkynylcarbonyl, C₁₋₇ alkoxycarbonyl, C₃₋₇ cycloalkylcarbonyl, 3- to 8-membered heterocycloalkylcarbonyl, C₁₋₇ alkyl S(O)₂-, C₂₋₇ alkenyl S(O)₂-, C₂₋₇ alkynyl S(O)₂-, C₁₋₇ alkoxy S(O)₂-, C₃₋₇ cycloalkyl S(O)₂-, 3- to 8-membered heterocycloalkyl S(O)₂- are optionally substituted with one or more Rₑ substituents;
R_{c} and R_{d} are each independently selected from hydrogen, C₁₋₆ alkoxy, C₁₋₆ alkyl, C₂₋₆ alkenyl, C₂₋₆ alkynyl, C₃₋₆ cycloalkyl, and aminosulfonyl, or R_{c} and R_{d}, together with the connected N atom, form a 3- to 6-membered heterocyclic ring;
each Rₑ is the same as or different from each other and is independently selected from hydrogen, deuterium, tritium, halogen, amino, hydroxyl, cyano, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy.
n1 is an integer of 0, 1, 2, 3, or 4.
n2 is an integer of 1, 2, 3, or 4.

Preferably, R₁ is selected from a hydrogen atom, C₁₋₄ alkyl, C₃₋₆ cycloalkyl, C₆₋₁₀ aryl, 4-to 6-membered heterocyclyl containing 1 to 3 heteroatoms selected from N, O, and S, 5- to 6-membered heteroaryl containing 1 to 3 heteroatoms selected from N, O, and S, wherein the C₁₋₄ alkyl, C₃₋₆ cycloalkyl, C₆₋₁₀ aryl, 4- to 6-membered heterocyclyl, and 5- to 6-membered heteroaryl are optionally substituted with 1 to 3 Rₐ substituents;
each Rₐ is the same as or different from each other and is independently selected from hydrogen, deuterium, tritium, halogen, amino, hydroxyl, cyano, C₁₋₄ alkoxy, C₁₋₄ alkyl, C₃₋₆ cycloalkyl, 4- to 6-membered heterocycloalkyl containing 1 to 3 heteroatoms selected from N, O, and S.

Preferably, R₂ is selected from a hydrogen atom, a halogen atom, C₁₋₄ alkyl, C₂₋₄ alkenyl, C₂₋₄ alkynyl, C₁₋₄ alkoxy, C₃₋₆ cycloalkyl, C₆₋₁₀ aryl, 4- to 6-membered heterocyclyl containing 1 to 3 heteroatoms selected from N, O, and S, 5- to 6-membered heteroaryl containing 1 to 3 heteroatoms selected from N, O, and S, wherein the C₁₋₄ alkyl, C₂₋₄ alkenyl, C₂₋₄ alkynyl, C₁₋₄ alkoxy, C₃₋₆ cycloalkyl, C₆₋₁₀ aryl, 4- to 6-membered heterocyclyl, and 5- to 6-membered heteroaryl are optionally substituted with one or more R_{b} substituents,
each R_{b} is the same as or different from each other and is independently selected from hydrogen, deuterium, tritium, halogen, amino, hydroxyl, cyano, C₁₋₄ alkoxy, C₁₋₄ alkyl, C₂₋₄ alkenyl, C₂₋₄ alkynyl, C₃₋₆ cycloalkyl, 4- to 6-membered heterocycloalkyl containing 1 to 3 heteroatoms selected from N, O, and S.

Preferably, R₃ is selected from C₁₋₄ alkylcarbonyl, C₂₋₄ alkenylcarbonyl, C₂₋₄ alkynylcarbonyl, C₁₋₄ alkoxycarbonyl, C₃₋₆ cycloalkylcarbonyl, sulfonic acid group, aminosulfonyl, 3- to 6-membered heterocycloalkylcarbonyl containing 1 to 3 heteroatoms selected from N, O, and S, NR_{c}R_{d} carbonyl, C₁₋₄ alkyl S(O)₂-, C₂₋₄ alkenyl S(O)₂-, C₂₋₄ alkynyl S(O)₂-, C₁₋₄ alkoxy S(O)₂-, C₃₋₆ cycloalkyl S(O)₂-, 3- to 6-membered heterocycloalkyl S(O)₂- containing 1 to 3 heteroatoms selected from N, O, and S, NR_{c}R_{d}S(O)₂-, wherein the C₁₋₄ alkylcarbonyl, C₂₋₄ alkenylcarbonyl, C₂₋₄ alkynylcarbonyl, C₁₋₄ alkoxycarbonyl, C₃₋₆ cycloalkylcarbonyl, 3- to 6-membered heterocycloalkylcarbonyl, C₁₋₄ alkyl S(O)₂-, C₂₋₄ alkenyl S(O)₂-, C₂₋₄ alkynyl S(O)₂-, C₁₋₄ alkoxy S(O)₂-, C₃₋₆ cycloalkyl S(O)₂-, 3- to 6-membered heterocycloalkyl S(O)₂- are optionally substituted with 1 to 3 Rₑ substituents;
R_{c} and R_{d} are each independently selected from hydrogen, C₁₋₄ alkoxy, C₁₋₄ alkyl, C₂₋₄ alkenyl, C₂₋₄ alkynyl, C₃₋₆ cycloalkyl, aminosulfonyl, or R_{c} and R_{d}, together with the connected N atom, form a 3- to 6-membered heterocyclic ring;
each Rₑ is the same as or different from each other and is independently selected from hydrogen, deuterium, tritium, halogen, amino, hydroxyl, cyano, C₁₋₄ alkoxy, C₁₋₄ alkyl, C₂₋₄ alkenyl, C₂₋₄ alkynyl, C₃₋₆ cycloalkyl, 4- to 6-membered heterocycloalkyl containing 1 to 3 heteroatoms selected from N, O, and S.

Preferably, R₁ is selected from a hydrogen atom, C₁₋₃ alkyl, C₃₋₆ cycloalkyl, 4- to 6-membered heterocyclyl containing 1 or 2 heteroatoms selected from N, O, and S, 5- to 6-membered heteroaryl containing 1 or 2 heteroatoms selected from N, O, and S, wherein the C₁₋₃ alkyl, C₃₋₆ cycloalkyl, 4- to 6-membered heterocyclyl, and 5- to 6-membered heteroaryl are optionally substituted with 1 or 2 Rₐ substituents;
each Rₐ is the same as or different from each other and is independently selected from hydrogen, deuterium, tritium, halogen, amino, hydroxyl, cyano, C₁₋₃ alkoxy, C₁₋₃ alkyl, C₃₋₆ cycloalkyl;
R₂ is selected from a hydrogen atom, a halogen atom, C₁₋₃ alkyl, C₂₋₃ alkenyl, C₂₋₃ alkynyl, C₁₋₃ alkoxy, C₃₋₆ cycloalkyl, C₆₋₁₀ aryl, 4- to 6-membered heterocyclyl containing 1 or 2 heteroatoms selected from N, O, and S, 5- to 6-membered heteroaryl containing 1 or 2 heteroatoms selected from N, O, and S, wherein the C₁₋₃ alkyl, C₂₋₃ alkenyl, C₂₋₃ alkynyl, C₁₋₃ alkoxy, C₃₋₆ cycloalkyl, C₆₋₁₀ aryl, 4- to 6-membered heterocyclyl, and 5- to 6-membered heteroaryl are optionally substituted with one or more R_{b} substituents,
each R_{b} is the same as or different from each other and is independently selected from hydrogen, deuterium, tritium, halogen, amino, hydroxyl, cyano, C₁₋₃ alkoxy, C₁₋₃ alkyl, C₂₋₃ alkenyl, C₂₋₃ alkynyl, C₃₋₆ cycloalkyl;
R₃ is selected from C₁₋₃ alkylcarbonyl, C₂₋₃ alkenylcarbonyl, C₂₋₃ alkynylcarbonyl, C₁₋₃ alkoxycarbonyl, C₃₋₆ cycloalkylcarbonyl, sulfonic acid group, aminosulfonyl, 3- to 6-membered heterocycloalkylcarbonyl containing 1 or 2 heteroatoms selected from N, O, and S, NR_{c}R_{d} carbonyl, C₁₋₃ alkyl S(O)₂-, C₂₋₃ alkenyl S(O)₂-, C₂₋₃ alkynyl S(O)₂-, C₁₋₃ alkoxy S(O)₂-, C₃₋₆ cycloalkyl S(O)₂-, 3- to 6-membered heterocycloalkyl S(O)₂- containing 1 or 2 heteroatoms selected from N, O, and S, NR_{c}R_{d}S(O)₂-, wherein the C₁₋₃ alkylcarbonyl, C₂₋₃ alkenylcarbonyl, C₂₋₃ alkynylcarbonyl, C₁₋₃ alkoxycarbonyl, C₃₋₆ cycloalkylcarbonyl, 3- to 6-membered heterocycloalkylcarbonyl, C₁₋₃ alkyl S(O)₂-, C₂₋₃ alkenyl S(O)₂-, C₂₋₃ alkynyl S(O)₂-, C₁₋₃ alkoxy S(O)₂-, C₃₋₆ cycloalkyl S(O)₂-, 3- to 6-membered heterocycloalkyl S(O)₂- are optionally substituted with 1 or 2 Rₑ substituents; and
R_{c} and R_{d} are each independently selected from hydrogen, C₁₋₄ alkoxy, C₁₋₄ alkyl, C₃₋₆ cycloalkyl, aminosulfonyl, or R_{c} and R_{d}, together with the connected N atom, form a 3- to 6-membered heterocyclic ring;
each Rₑ is the same as or different from each other and is independently selected from hydrogen, deuterium, tritium, halogen, amino, hydroxyl, cyano, C₁₋₃ alkoxy, C₁₋₃ alkyl, C₃₋₆ cycloalkyl.

Preferably, R₁ is selected from a hydrogen atom, methyl, ethyl, n-propyl, isopropyl, cyclopropyl, deuterated methyl, deuterated ethyl, deuterated n-propyl, deuterated isopropyl, deuterated cyclopropyl.

Preferably, R₂ is selected from a hydrogen atom, fluorine, chlorine, bromine, methyl, ethyl, n-propyl, isopropyl, monofluoromethyl, difluoromethyl, trifluoromethyl, dichloromethyl, trichloromethyl, monofluoroethyl, difluoroethyl, trifluoroethyl, tetrafluoroethyl, pentafluoroethyl, dichloroethyl, trichloroethyl, tetrachloroethyl, pentachloroethyl, difluoropropyl, trifluoropropyl, tetrafluoropropyl, pentafluoropropyl, hexafluoropropyl, perfluoropropyl, monochloropropyl, dichloropropyl, trichloropropyl, tetrachloropropyl, pentachloropropyl, hexachloropropyl, perchloropropyl.

Preferably, R₃ is selected from a hydrogen atom, methyl-S(O)₂-, ethyl-S(O)₂-, n-propylS(O)₂-, isopropyl-S(O)₂-, cyclopropyl-S(O)₂-, oxiranyl-S(O)₂-, cyclobutyl-S(O)₂-, oxetanyl-S(O)₂-, methoxy-S(O)₂-, ethoxy-S(O)₂-, n-propoxy-S(O)₂-, isopropoxy-S(O)₂-, cyclopropoxy-S(O)₂-, oxiranyloxy-S(O)₂-, cyclobutoxy-S(O)₂-, oxetanyloxy-S(O)₂-, N,N-dimethylamino-S(O)₂-, trifluoromethyl-S(O)₂-, amino-S(O)₂-, SO₃H-, pyrroline-1-S(O)₂-, piperidine-1-S(O)₂-, morpholine-1-S(O)₂-, methylcarbonyl, ethylcarbonyl, n-propylcarbonyl, isopropylcarbonyl, cyclopropylcarbonyl, oxiranylcarbonyl, cyclobutylcarbonyl, oxetanylcarbonyl, methoxycarbonyl, ethoxycarbonyl, n-propoxycarbonyl, isopropoxycarbonyl, cyclopropoxycarbonyl, oxiranyloxycarbonyl, cyclobutoxycarbonyl, oxetanyloxycarbonyl, N,N-dimethylaminocarbonyl, trifluoromethylcarbonyl, and 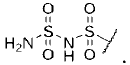
preferably, n1 is 1.
Preferably, n2 is 1.

Preferably, the compound represented by general formula (I), or the stereoisomer, tautomer, deuterated derivative or pharmaceutically acceptable salt thereof is one of the following compounds:

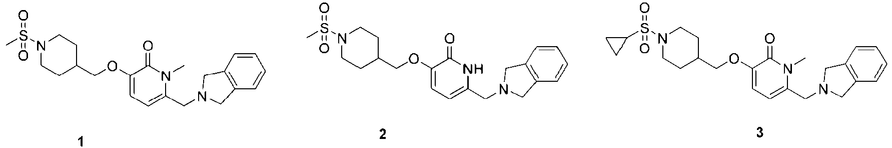

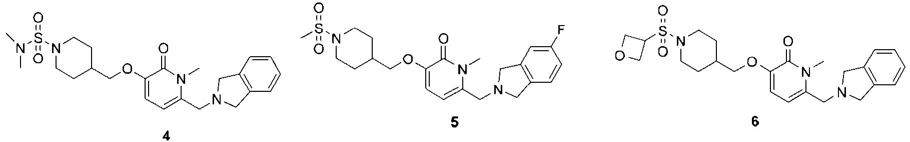

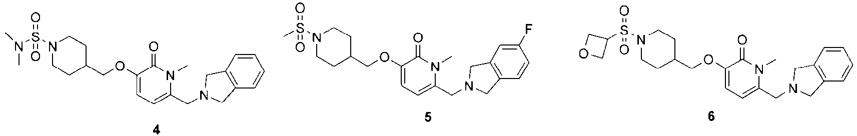

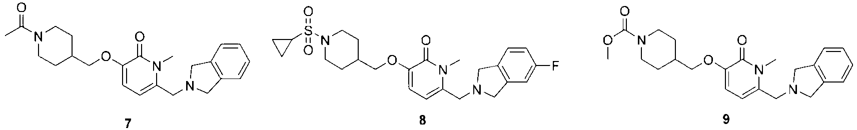

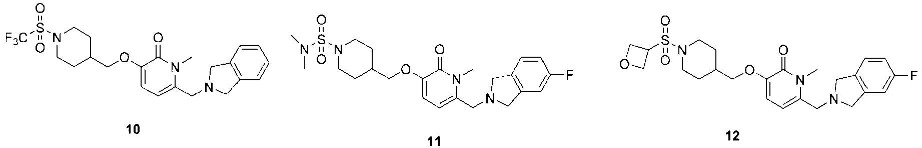

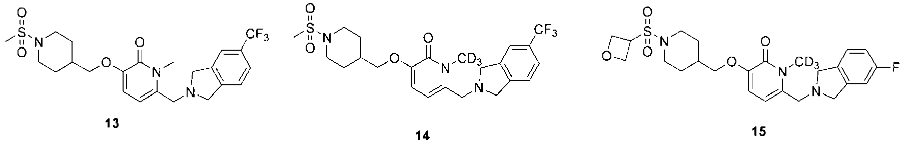

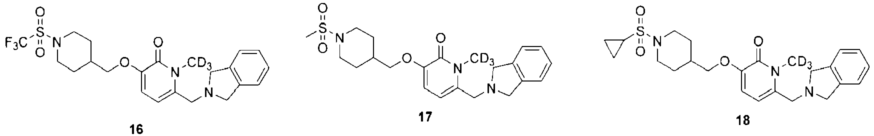

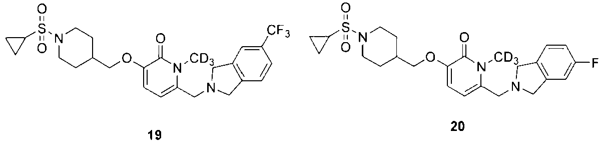

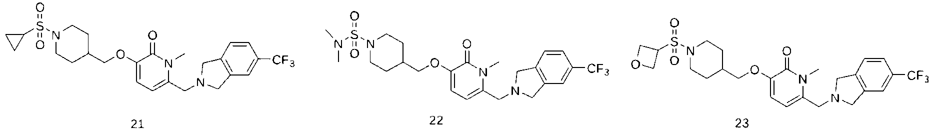

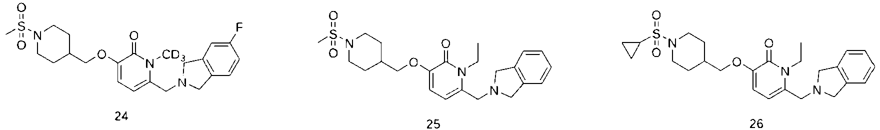

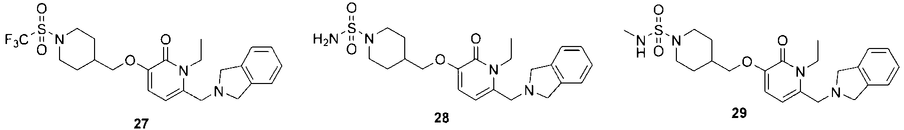

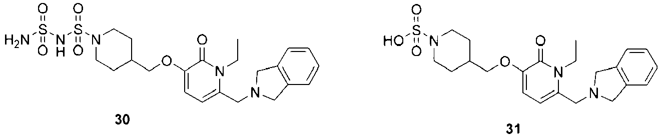

According to another aspect of the present disclosure, another object of the present disclosure is to provide a method for preparing the compound represented by general formula (I), or a stereoisomer, tautomer, deuterated derivative or pharmaceutically acceptable salt thereof, the method comprising the following steps: 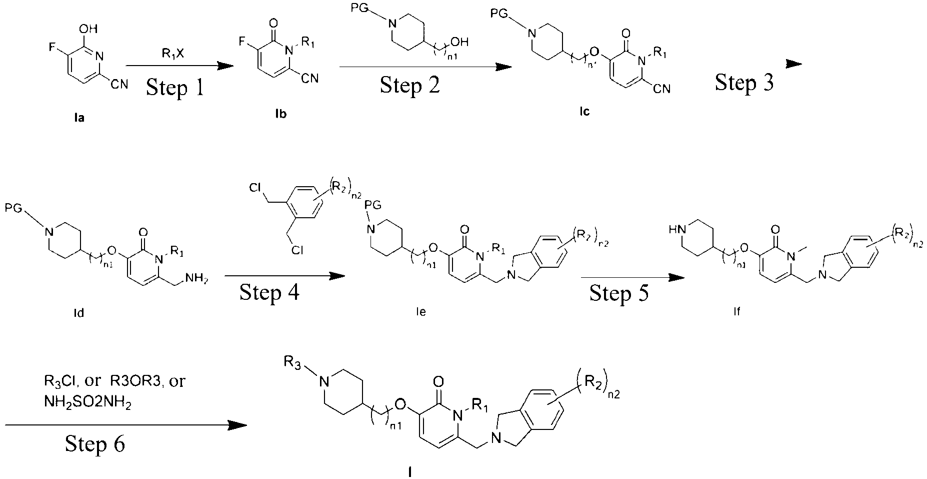
step 1) subjecting a commercialized compound (Ia) and a halogenated compound R₁X to a substitution addition reaction to obtain a compound represented by general formula (Ib);
step 2) subjecting the compound represented by general formula (Ib) and a piperidine compound 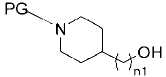 with a hydroxyalkyl substituent to a substitution reaction to obtain a compound represented by general formula (Ic);
step 3) reducing cyano in the compound represented by general formula (Ic) to obtain a compound represented by general formula (Id) with corresponding amino;
step 4) subjecting the compound represented by general formula (Id) and a dibenzyl chloride compound containing R₂ substituent to a cyclization reaction to obtain a compound represented by general formula (Ie);
step 5) removing an amino protecting group (PG) from the compound represented by general formula (Ie) to obtain a compound represented by general formula (If);
step 6) reacting the compound represented by general formula (If) with R₃Cl or R₃OR₃, or NH₂SO₂NH₂ to obtain a compound represented by general formula (I);
wherein:
   PG is an amino protecting group, selected from benzyloxycarbonyl (Cbz), tert-butoxycarbonyl (Boc), fluorenylmethoxycarbonyl (Fmoc), p-methoxybenzyl (PMB), benzyl (Bn), trityl (Trt), p-toluenesulfonyl (Tos), phthaloyl (Pht), allyloxycarbonyl (Alloc).

The substituents R₁, R₂, R₃, n1, and n2 are defined as in general formula (I).

According to another aspect of the present disclosure, the present disclosure provides the use of a compound represented by general formula (I), or a stereoisomer, tautomer, deuterated derivative or pharmaceutically acceptable salt thereof as a CYP11A1 inhibitor.

According to another aspect of the present disclosure, the present disclosure provides the use of the compound represented by general formula (I), or the stereoisomer, tautomer, deuterated derivative or pharmaceutically acceptable salt thereof in the preparation of a drug for treating a steroid hormone-dependent disease.

Preferably, the steroid hormone-dependent disease is cancer.

According to another aspect of the present disclosure, the present disclosure provides the use of the compound represented by general formula (I), or the stereoisomer, tautomer, deuterated derivative or pharmaceutically acceptable salt thereof in the preparation of a drug for treating a steroid receptor-dependent disease such as prostate cancer or breast cancer.

According to another aspect of the present disclosure, the present disclosure provides a pharmaceutical composition, the pharmaceutical composition comprises a therapeutically effective amount of the compound represented by general formula (I), or the stereoisomer, tautomer, deuterated derivative or pharmaceutically acceptable salt thereof according to the present disclosure as an active ingredient, and a pharmaceutically acceptable excipient.

According to another aspect of the present disclosure, the present disclosure provides a kit for treating prostate cancer or breast cancer, which comprises:
the compound represented by general formula (I), or the stereoisomer, tautomer, deuterated derivative or pharmaceutically acceptable salt thereof according to the present disclosure, or the pharmaceutical composition comprising the compound represented by general formula (I), or the stereoisomer, tautomer, deuterated derivative or pharmaceutically acceptable salt thereof according to the present disclosure as an active ingredient; and instructions for using the compound or the pharmaceutical composition.

The kit described herein may comprise a single dose or multiple doses of the compound or pharmaceutical composition. The kit can be used for the method of the present disclosure. In certain embodiments, the kit further comprises instructions for using the compound or pharmaceutical composition.

According to another aspect of the present disclosure, the present disclosure provides a method for treating prostate cancer, the method comprises administering a therapeutically effective amount of the compound represented by general formula (I), or the stereoisomer, tautomer, deuterated derivative or pharmaceutically acceptable salt thereof according to the present disclosure, or the pharmaceutical composition according to the present disclosure to a prostate cancer patient in need thereof.

According to another aspect of the present disclosure, the present disclosure provides a method for treating breast cancer, the method comprises administering a therapeutically effective amount of the compound represented by general formula (I), or the stereoisomer, tautomer, deuterated derivative or pharmaceutically acceptable salt thereof according to the present disclosure, or the pharmaceutical composition according to the present disclosure to a breast cancer patient in need thereof.

According to another aspect of the present disclosure, the present disclosure provides a method for administering the compound represented by general formula (I), or the stereoisomer, tautomer, deuterated derivative or pharmaceutically acceptable salt thereof, wherein the compound can be administered together with at least one selected from glucocorticoids and mineralocorticoids.

According to another aspect of the present disclosure, the present disclosure provides a method for administering the compound represented by general formula (I), or the stereoisomer, tautomer, deuterated derivative or pharmaceutically acceptable salt thereof, wherein the compound can be administered together with one or more other anticancer drugs, the anticancer drugs are selected from at least one of a nonsteroidal androgen receptor antagonist, a steroid synthesis inhibitor, a chemotherapeutic agent, an estrogen receptor antagonist.

### Beneficial Effects

Compared with the prior art, the compound of the present disclosure has a new skeleton. The compound of the present disclosure unexpectedly shows an inhibitory effect on the biosynthesis of pregnenolone and testosterone. Since the pregnenolone is catalytically synthesized by CYP11A1, the compound shows a good inhibitory effect on CYP11A. Clinically, the compound can be used for treating steroid receptor-dependent associated diseases, especially steroid receptor-dependent cancers, such as prostate cancer and breast cancer.

### DETAILED DESCRIPTION OF EMBODIMENTS

Hereinafter, the present disclosure will be described in detail. Before description, it should be understood that the terms used in the description and the appended claims should not be interpreted as being limited to general meanings and dictionary meanings, but should be interpreted according to the meanings and concepts corresponding to the technical aspects of the present disclosure on the basis of the principle of allowing the inventors to properly define terms for the best interpretation. Therefore, the description presented here is preferred examples for the illustrative purpose only and is not intended to limit the scope of the present disclosure, so it should be understood that other equivalents or improvements can be obtained therefrom without departing from the spirit and scope of the present disclosure.

Herein, the terms "comprise", "include", "have", "contain", or any other similar terms are all open-ended transitional phrases, which are intended to cover non-exclusive inclusions. For example, a composition or article containing plural elements is not limited to these elements listed herein but may also include other elements that are not explicitly listed but are usually inherent in the composition or article. Besides, unless explicitly stated to the contrary, the term "or" refers to an inclusive "or" rather than an exclusive "or". For example, the condition "A or B" is satisfied in any of the following cases: A is true (or exists) and B is false (or does not exist), A is false (or does not exist) and B is true (or exists), and both A and B are true (or exist). In addition, the interpretation of the terms "comprise", "include", "have", and "contain" herein should be regarded as having been specifically disclosed and covering closed or semi-closed conjunctions such as "consisting of ..." and "substantially consisting of ...".

Herein, all characteristics or conditions defined in the form of numerical ranges or percentage ranges are only for simplicity and convenience. Accordingly, the description of the numerical range or percentage range should be regarded as covering and specifically disclosing all possible sub-ranges and individual values within the range, especially integer values. For example, the description of the range of "1 to 8" should be regarded as having specifically disclosed all sub-ranges, such as 1 to 7, 2 to 8, 2 to 6, 3 to 6, 4 to 8, and 3 to 8, especially sub-ranges defined by all integer values, and should be regarded as having specifically disclosed individual values in the range, such as 1, 2, 3, 4, 5, 6, 7, and 8. Unless otherwise specified, the foregoing interpretation method is applicable to the entire content of the full text of the present disclosure, regardless of whether the scope is wide.

If quantities or other numerical values or parameters are expressed as ranges, preferred ranges, or a series of upper and lower limits, it should be understood that all ranges formed by any pair of upper or preferred values of the range and lower or preferred values of the range have been specifically disclosed herein, regardless of whether these ranges are disclosed separately. In addition, when a numerical value range is mentioned herein, unless otherwise specified, the range shall include the endpoints thereof and all integers and fractions within the range.

Herein, a numerical value should be understood as having the precision of the significant digits of the numerical value on the premise that the objects of the present disclosure can be achieved. For example, the number 40.0 should be understood to cover a range of from 39.50 to 40.49.

Herein, in the case of using Markush group or alternative language to describe the features or examples of the present disclosure, those skilled in the art should understand that subgroups or any individual elements of all elements within the Markush group or alternative language can also be used to describe the present disclosure. For example, if X is described as being "selected from the group consisting of X1, X2, and X3", it also means that the statement of X being X1 and/or the statement of X being X1 and/or X2 have been fully described. Furthermore, in the case of using Markush group or alternative language to describe the features or examples of the present disclosure, those skilled in the art should understand that any combination of subgroups or individual elements of all elements within the Markush group or alternative language can also be used to describe the present disclosure. Accordingly, for example, if X is described as being "selected from the group consisting of X1, X2, and X3" and Y is described as being "selected from the group consisting of Y1, Y2, and Y3", it means that the statement of X being X1 or X2 or X3 and Y being Y1 or Y2 or Y3 has been fully described.

### Definitions

Definitions of specific functional groups and chemical terms are described in more detail below. Chemical elements are determined according to the periodic table of elements on the inner cover of CAS edition, Handbook of Chemistry and Physics, 75th edition, and specific functional groups are usually defined as described therein. In addition, the general principles of organic chemistry and specific functional moieties and reactivity are described in the following books: Thomas Sorrell, Organic Chemistry, University Science Books, Sausalito, 1999; Smith and March, March's Advanced Organic Chemistry, 5th Edition, John Wiley&Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. The present disclosure is not intended to be limited in any way by the exemplary list of substituents described herein.

The compounds described herein may comprise one or more asymmetric centers and may thus exist in various isomeric forms, such as enantiomers and/or diastereomers. For example, the compounds described herein may be in the form of individual enantiomers, diastereomers, or geometric isomers, or may be in the form of stereoisomer mixtures, including racemic mixtures and mixtures rich in one or more stereoisomers. Isomers can be separated from the mixture by methods known to those skilled in the art, including chiral high-pressure liquid chromatography (HPLC) and the formation and crystallization of chiral salts; or preferably, isomers can be prepared by asymmetric synthesis. For example, see Jacques et al., Enantiomers, Racemates and Resolutions (Wiley Interscience, New York, 1981); Wilen et al., Tetrahedron 33: 2725 (1977); Eliel, Stereochemistry of Carbon Compounds (McGraw-Hill, NY, 1962); and Wilen, Tables of Resolving Agents and Optical Resolutions, page 268 (edited by E. L. Eliel, The University of Notre Dame Press, Notre Dame de Paris, IN 1972). The present disclosure additionally encompasses that the compounds described herein are individual isomers substantially free of other isomers, or are mixtures of various isomers.

When a series of values are listed, it is intended to cover every value and subrange within the range. For example, "C₁₋₆" is intended to cover C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "alkyl" refers to a linear or branched saturated hydrocarbon group having 1 to 7 carbon atoms ("C₁₋₇ alkyl"). In some embodiments, the alkyl has 1 to 7 carbon atoms ("C₁₋₇ alkyl"). In some embodiments, the alkyl has 1 to 6 carbon atoms ("C₁₋₆ alkyl"). In some embodiments, the alkyl has 1 to 5 carbon atoms ("C₁₋₅ alkyl"). In some embodiments, the alkyl has 1 to 4 carbon atoms ("C₁₋₄ alkyl"). In some embodiments, the alkyl has 1 to 3 carbon atoms ("C₁₋₃ alkyl"). In some embodiments, the alkyl has 1 to 2 carbon atoms ("C₁₋₂ alkyl"). In some embodiments, the alkyl has 1 carbon atom ("C₁ alkyl"). In some embodiments, the alkyl has 2 to 6 carbon atoms ("C₂₋₆ alkyl"). Examples of C₁₋₆ alkyl include methyl (C₁), ethyl (C₂), propyl (C₃) (e.g., n-propyl and isopropyl), butyl (C₄) (e.g., n-butyl, tert-butyl, sec-butyl, isobutyl), pentyl (C₅) (e.g., n-pentyl, 3-pentyl, neopentyl, 3-methyl-2-butyl, tert-pentyl), and hexyl (C₆) (e.g., n-hexyl). Further examples of alkyl include n-heptyl (C₇), etc. Unless otherwise specified, each example of alkyl is independently unsubstituted or substituted with one or more substituents (e.g., halogen, such as F). In certain embodiments, the alkyl is unsubstituted C₁₋₆ alkyl, such as -CH₃. In certain embodiments, the alkyl is substituted C₁₋₆ alkyl, such as -CF₃.

"Alkoxy" represents monovalent □O□ alkyl, wherein the alkyl moiety has a specified number of carbon atoms. The alkoxy in the present disclosure usually contains 1□6 carbon atoms ("C1-6 alkoxy"), for example, including methoxy, ethoxy, isopropoxy, tert-butoxy, etc. Unless otherwise specified, each example of alkoxy is independently and optionally substituted, i.e., unsubstituted ("unsubstituted alkoxy") or substituted with one or more substituents ("substituted alkoxy"). In certain embodiments, the alkoxy is unsubstituted C₁₋₆ alkoxy. In certain embodiments, the alkoxy is substituted C₁₋₆ alkoxy.

"Alkenyl" refers to a linear or branched hydrocarbon group having 2 to 7 carbon atoms with one or more carbon-carbon double bonds and without triple bonds ("C₂₋₇ alkenyl"). In some embodiments, the alkenyl has 2 to 7 carbon atoms ("C₂₋₇ alkenyl"). In some embodiments, the alkenyl has 2 to 6 carbon atoms ("C₂₋₆ alkenyl"). In some embodiments, the alkenyl has 2 to 5 carbon atoms ("C₂₋₅ alkenyl"). In some embodiments, the alkenyl has 2 to 4 carbon atoms ("C₂₋₄ alkenyl"). In some embodiments, the alkenyl has 2 to 3 carbon atoms ("C₂₋₃ alkenyl"). In some embodiments, the alkenyl has 2 carbon atoms ("C₂ alkenyl"). The one or more carbon-carbon double bonds may be internal (e.g., in 2-butenyl) or at the end (e.g., in 1-butenyl). Examples of C₂₋₄ alkenyl include vinyl (C₂), 1-propenyl (C₃), 2-propenyl (C₃), 1-butenyl (C₄), 2-butenyl (C₄), butadienyl (C₄), etc. Examples of C₂₋₆ alkenyl include the above-mentioned C₂₋₄ alkenyl and pentenyl (C₅), pentadienyl (C₅), hexenyl (C₆), etc. Further examples of alkenyl include heptenyl (C₇), etc. Unless otherwise specified, each example of alkenyl is independently optionally substituted, i.e., unsubstituted or substituted with one or more substituents. In certain embodiments, in the alkenyl, the C=C double bond without the stereochemistry being specified can be an (E)- or (Z)- double bond.

"Alkynyl" refers to a linear or branched hydrocarbon group having 2 to 7 carbon atoms with one or more carbon-carbon triple bonds and optionally one or more double bonds ("C₂₋₇ alkynyl"). In some embodiments, the alkynyl has 2 to 7 carbon atoms ("C₂₋₇ alkynyl"). In some embodiments, the alkynyl has 2 to 6 carbon atoms ("C₂₋₆ alkynyl"). In some embodiments, the alkynyl has 2 to 5 carbon atoms ("C₂₋₅ alkynyl"). In some embodiments, the alkynyl has 2 to 4 carbon atoms ("C₂₋₄ alkynyl"). In some embodiments, the alkynyl has 2 to 3 carbon atoms ("C₂₋₃ alkynyl"). In some embodiments, the alkynyl has 2 carbon atoms ("C₂ alkynyl"). The one or more carbon-carbon triple bonds may be internal (e.g., in 2-butynyl) or at the end (e.g., in 1-butynyl). Examples of C₂₋₄ alkynyl include, but are not limited to, ethynyl (C₂), 1-propynyl (C₃), 2-propynyl (C₃), 1-butynyl (C₄), 2-butynyl (C₄), etc. Examples of C₂₋₆ alkenyl include the above-mentioned C₂₋₄ alkynyl, and pentynyl (C₅), hexynyl (C₆), etc. Further examples of alkynyl include heptylynyl, etc. Unless otherwise specified, each example of alkynyl is independently optionally substituted, i.e., unsubstituted or substituted with one or more substituents.

"Cycloalkyl" refers to a non-aromatic cyclic hydrocarbon group having 3 to 6 ring carbon atoms ("C₃₋₆ cycloalkyl") and zero heteroatoms in the non-aromatic ring system. Exemplary C₃₋₆ cycloalkyl include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), etc. As shown in the previous examples, in certain embodiments, the cycloalkyl is a monocyclic ring ("monocyclic cycloalkyl") or contains a fused, bridged, or spirocyclic ring system, such as a bicyclic system ("bicyclic cycloalkyl") and may be saturated or partially unsaturated. "Cycloalkyl" also includes a ring system in which the cycloalkyl as defined above is fused with one or more aryl or heteroaryl groups, wherein the point of attachment is on the carbocyclic ring, and in this case, the number of carbons still refers to the number of carbons in the carbocyclic ring system. Unless otherwise specified, each example of cycloalkyl is independently optionally substituted, i.e., unsubstituted or substituted with one or more substituents.

"Heterocycloalkyl" refers to a group with a 4- to 8-membered non-aromatic ring system having ring carbon atoms and 1 to 3 ring heteroatoms, wherein each heteroatom is independently selected from nitrogen, oxygen, sulfur, boron, phosphorus, and silicon ("4- to 8-membered heterocyclyl"). In heterocyclyl containing one or more nitrogen atoms, the point of attachment can be a carbon atom or a nitrogen atom as long as the valence allows. The heterocycloalkyl may be a monocyclic ring ("monocyclic heterocycloalkyl") or a fused, bridged, or spirocyclic ring system, such as a bicyclic ring ("bicyclic heterocycloalkyl"), and may be saturated or partially unsaturated. A heterocycloalkyl bicyclic system may comprise one or more heteroatoms in one or two rings. "Heterocycloalkyl" also includes a ring system in which the heterocyclic ring as defined above is fused with one or more carbocyclic groups, wherein the point of attachment is on the carbocyclic group or heterocyclic ring, or in which the heterocyclic ring as defined above is fused with one or more aryl or heteroaryl groups, wherein the point of attachment is on the heterocyclic ring, and in this case, the number of ring members still refers to the number of ring members in a heterocyclic ring system. Unless otherwise specified, each example of heterocyclyl is independently optionally substituted, i.e., unsubstituted or substituted with one or more substituents.

Exemplary 3-membered heterocyclyl containing one heteroatom includes, but are not limited to, azirdinyl, oxiranyl, thiiranyl. Exemplary 4-membered heterocyclyl containing one heteroatom includes, but are not limited to, azetidinyl, oxetanyl, and thietanyl. Exemplary 5-membered heterocyclyl containing one heteroatom includes, but are not limited to, tetrahydrofuryl, dihydrofuryl, tetrahydrothienyl, dihydrothienyl, pyrrolidinyl, dihydropyrrolyl, and pyrrolyl-2,5-dione. Exemplary 5-membered heterocyclyl containing two heteroatoms includes, but are not limited to, dioxolanyl, oxasulfuranyl, disulfuranyl, and oxazolidin-2-one. Exemplary 5-membered heterocyclyl containing three heteroatoms includes, but are not limited to, triazolinyl, oxadiazolinyl, and thiadiazolinyl. Exemplary 6-membered heterocyclyl containing one heteroatom includes, but are not limited to, piperidinyl, tetrahydropyranyl, dihydropyridinyl, and thianyl. Exemplary 6-membered heterocyclyl containing two heteroatoms includes, but are not limited to, piperazinyl, morpholinyl, dithianyl, and dioxanyl. Exemplary 6-membered heterocyclyl containing two heteroatoms includes, but are not limited to, triazinyl. Exemplary groups in which 5-membered heterocycloalkyl is fused with a C₆ aryl ring (also referred to herein as 5,6-bicyclic heterocyclic ring) include, but are not limited to, dihydroindolyl, isodihydroindolyl, dihydrobenzofuryl, dihydrobenzothienyl, benzoxazolinonyl, etc. Exemplary groups in which 6-membered heterocycloalkyl is fused with an aromatic ring (also referred to herein as 6,6-bicyclic heterocyclic ring) include, but are not limited to, tetrahydroquinolinyl, tetrahydroisoquinolinyl, etc.

"Aryl" refers to a group ("C₆₋₁₄ aryl") having a monocyclic or polycyclic (e.g., bicyclic or tricyclic) 4n+2 aromatic ring system (e.g., having 6, 10, or 14 π electrons shared in a cyclic array) with 6-14 ring carbon atoms and zero heteroatoms provided in the aromatic ring system. In some embodiments, the aryl has 6 ring carbon atoms ("C₆ aryl"; e.g., phenyl). In some embodiments, the aryl has 10 ring carbon atoms ("C₁₀ aryl"; e.g., naphthyl, such as 1-naphthyl and 2-naphthyl). In some embodiments, the aryl has 14 ring carbon atoms ("C₁₄ aryl"; e.g., anthryl). "Aryl" also includes a ring system in which the aryl ring as defined above is fused with one or more carbocyclic groups or heterocyclic groups, wherein the group or the point of attachment is on the aromatic ring, and in this case, the number of carbon atoms still refers to the number of carbon atoms in the aromatic ring system. Unless otherwise specified, each example of aryl is independently and optionally substituted, i.e., unsubstituted ("unsubstituted aryl") or substituted with one or more substituents ("substituted aryl"). In certain embodiments, the aryl is unsubstituted C₆₋₁₄ aryl. In certain embodiments, the aryl is substituted C₆₋₁₄ aryl.

"Heteroaryl" refers to a group with a 5- to 8-membered monocyclic or bicyclic 4n+2 aromatic ring system with ring carbon atoms and 1-4 ring heteroatoms provided in the aromatic ring system (for example, with 6 π electrons shared in a ring array), wherein each heteroatom is independently selected from nitrogen, oxygen, and sulfur ("5- to 8-membered heteroaryl"). In heteroaryl containing one or more nitrogen atoms, the point of attachment can be a carbon atom or a nitrogen atom as long as the valence allows. A heteroaryl bicyclic system may comprise one or more heteroatoms in one or two rings. "Heteroaryl" includes a ring system in which the heteroaryl ring as defined above is fused with one or more carbocyclic groups or heterocyclic groups, wherein the point of attachment is on the heteroaryl ring, and in this case, the number of ring members still refers to the number of ring members in the heteroaryl ring system. "Heteroaryl" also includes a ring system in which a heteroaryl ring as defined above is fused with one or more aryl, wherein the point of attachment is on the aryl or the heteroaryl ring, and in this case, the number of ring members refers to the number of ring members in the fused (aryl/heteroaryl) ring system.

Exemplary 5-membered heteroaryl containing one heteroatom includes, but are not limited to, pyrrolyl, furyl, and thienyl. Exemplary 5-membered heteroaryl containing two heteroatoms includes, but are not limited to, imidazolyl, pyrazolyl, oxazolyl, isoxazolyl, thiazolyl, and isothiazolyl. Exemplary 5-membered heteroaryl containing three heteroatoms includes, but are not limited to, triazolyl, oxadiazolyl, and thiadiazolyl. Exemplary 5-membered heteroaryl containing four heteroatoms includes, but are not limited to, tetrazolyl. Exemplary 6-membered heteroaryl containing one heteroatom includes, but are not limited to, pyridyl. Exemplary 6-membered heteroaryl containing two heteroatoms includes, but are not limited to, pyridazinyl, pyrimidinyl, and pyrazinyl. Exemplary 6-membered heteroaryl containing three or four heteroatoms includes, but are not limited to, triazinyl and tetrazinyl respectively. Exemplary 7-membered heteroaryl containing one heteroatom includes, but are not limited to, azepinyl, oxepinyl, and thiepinyl. Exemplary 5,6-bicyclic heteroaryl includes, but are not limited to, indolyl, isoindolyl, indazolyl, benzotriazolyl, benzothienyl, isobenzothienyl, benzofuryl, benzisofuryl, benzimidazolyl, benzoxazolyl, benzoisoxazolyl, benzoxadiazolyl, benzothiazolyl, benzisothiazolyl, benzothiadiazolyl, indolizinyl, and purinyl. Exemplary 6,6-bicyclic heteroaryl includes, but are not limited to, naphthyridinyl, pteridinyl, quinolinyl, isoquinolinyl, cinnolinyl, quinoxalinyl, phthalazinyl, and quinazolinyl.

Unless explicitly provided otherwise, the atoms, moieties or groups described herein may be unsubstituted or substituted as long as valence permits.

"Halo" or "halogen" refers to fluorine (fluoro, -F), chlorine (chloro, -Cl), bromine (bromo, -Br), or iodine (iodo, -I).

In certain embodiments, a substituent present on a nitrogen atom is a nitrogen protecting group (also referred to as an amino protecting group). The nitrogen protecting groups are well known in the art and include those described in detail in Protecting Groups in Organic Synthesis, T. W. Greene and P. G. M. Wuts, 3rd edition, John Wiley&Sons, 1999, which is hereby incorporated herein by reference. For example, the amino protecting group may be selected from benzyloxycarbonyl (Cbz), tert-butoxycarbonyl (Boc), fluorenylmethoxycarbonyl (Fmoc), p-methoxybenzyl (PMB), benzyl (Bn), trityl (Trt), p-toluenesulfonyl (Tos), phthaloyl (Pht), allyloxycarbonyl (Alloc).

The term "pharmaceutically acceptable salt" refers to a salt that is suitable for use in contact with tissues of humans and lower animals within reasonable medical judgment without undue toxicity, irritation, allergic reaction, etc., and is commensurate with a rational benefit/risk ratio. Pharmaceutically acceptable salts are well known in the art. Examples are pharmaceutically acceptable salts described in detail by Berge et al. in J. Pharmaceutical Sciences, 1977,66,1-19, which is incorporated herein by reference. The pharmaceutically acceptable salts of the compounds described herein include salts derived from suitable inorganic and organic acids and bases. Examples of pharmaceutically acceptable nontoxic acid addition salts are amino salts formed with inorganic acids (such as hydrochloric acid, hydrobromic acid, phosphoric acid, sulfuric acid, and perchloric acid) or organic acids (acetic acid, oxalic acid, maleic acid, tartaric acid, citric acid, succinic acid, or malonic acid) or by using other methods known in the art (such as ion exchange). Other pharmaceutically acceptable salts include adipate, alginate, ascorbate, aspartate, benzenesulfonate, benzoate, bisulfate, borate, butyrate, camphorate, camphorsulfonate, citrate, cyclopentylpropionate, digluconate, dodecylsulfate, ethanesulfonate, formate, fumarate, glucoheptonate, glycerophosphate, gluconate, hemisulfate, heptanate, caproate, hydrogen iodide, 2-hydroxy-ethanesulfonate, lactobionate, lactate, laurate, dodecylsulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate, etc. Salts derived from suitable bases include alkali metal, alkaline earth metal, ammonium, and N⁺(C₁₋₄ alkyl)₄⁻ salts. Representative alkali or alkaline earth metal salts include sodium, lithium, potassium, calcium, magnesium, etc. Where appropriate, other pharmaceutically acceptable salts include nontoxic ammonium, quaternary ammonium and amine cations formed with counterions such as halide ions, hydroxide ions, carboxylate, sulfate, phosphate, nitrate, lower alkyl sulfonate, and aryl sulfonate.

The term "tautomer" or "tautomeric" refers to two or more compounds that interconvert from each other and are produced by at least one formal migration of a hydrogen atom and at least one change in valence (for example, a single bond becomes a double bond, a triple bond becomes a single bond, and vice versa). The exact proportion of the tautomers depends on several factors, including temperature, solvent, and pH. Tautomeric reactions (i.e., reactions that provide tautomeric pairs) can be catalyzed by acids or bases. Exemplary tautomerism reactions include ketone-enol, amide-imide, lactam-lactim, enamine-imine, and enamine-(different enamines) tautomerism reactions.

It should also be understood that compounds that have the same molecular formula but are different in properties, or the bonding order of the atoms thereof, or the spatial arrangement of the atoms thereof are termed "isomers". Isomers with different atomic spatial arrangements are termed "stereoisomers".

Stereoisomers that are not mirror images of each other are termed "diastereomers", and stereoisomers that are non-superimposable mirror images of each other are termed "enantiomers". When a compound has an asymmetric center, for example, it is bonded to four different groups, there may be a pair of enantiomers. Enantiomers can be characterized by the absolute configuration of the asymmetric center thereof, and described by the R- and S- order rules of Cahn and Prelog or described by the way in which the molecule rotates the plane of polarized light, and are expressed as dextrorotatory or levorotatory (i.e., (+) or (-)-isomers, respectively). Chiral compounds can exist as individual enantiomers or as mixtures thereof. A mixture containing equal proportions of enantiomers is termed "racemic mixture".

The term "inhibition" or "inhibitor" refers to the ability of a compound to reduce, slow down, hinder, or prevent the activity of a specific biological process (such as the activity of CYP11A1 enzyme in a cell relative to a carrier).

The "subject" intended for administration refers to a human (i.e., a male or female of any age group, such as a pediatric subject (such as an infant, a child, or an adolescent) or an adult subject (such as a young adult, a middle-aged human, or an elderly human). A "patient" refers to a human subject in need of a treatment for a disease.

The term "biological sample" is meant to include tissue samples (such as tissue sections and needle biopsy specimens of tissues); cell samples (such as cytological smears (such as Pap smears or blood smears) or cell samples obtained by microdissection); samples of whole organisms (such as yeast or bacterial samples); or any samples of cell parts, debris or organelles (obtained, for example, by lysing cells and separating components therefrom by centrifugation or other means). Other examples of biological samples include blood, serum, urine, semen, feces, cerebrospinal fluid, interstitial fluid, mucus, tears, sweat, pus, biopsy tissues (obtained, for example, by surgical biopsy or needle biopsy), nipple aspirates, milks, vaginal fluid, saliva, swabs (for example, oral swabs), or any materials containing biomolecules derived from the first biological sample.

The term "administration" refers to the introduction of the compound or the composition thereof described herein into or onto a subject by implantation, absorption, ingestion, injection, inhalation, or other means.

The term "treat" refers to reversing, alleviating, or delaying the onset of the disease described herein, or inhibiting the development of the disease described herein. In some embodiments, the treatment may be administered after one or more signs or symptoms of the disease have developed or have been observed. In other embodiments, the treatment can be administered where there are no signs or symptoms of the disease. For example, the treatment can be administered to susceptible subjects before the onset of symptoms (e.g., based on the history of the symptoms and/or based on the exposure to pathogens) to delay or prevent the occurrence of the disease. For example, in order to delay or prevent recurrence, the treatment may also be continued after the symptoms subside.

An "effective amount" of the compound as described herein refers to an amount sufficient to cause a desired biological response (i.e., to treat a condition). As will be understood by those of ordinary skill in the art, the effective amount of the compound described herein may vary depending on factors such as the desired biological endpoint, the pharmacokinetics of the compound, the condition being treated, the mode of administration, and the age and health of the subject. In certain embodiments, the effective amount is a therapeutically effective amount. In certain embodiments, the effective amount is for prophylactic treatment. In certain embodiments, the effective amount is the amount of the compound described herein in a single dose. In certain embodiments, the effective amount is the combined amount of the compound described herein in multiple doses.

A "therapeutically effective amount" of the compound described herein is an amount sufficient to provide therapeutic benefits in treating a condition or to delay or minimize one or more symptoms associated with the condition. The therapeutically effective amount of the compound refers to the amount of a therapeutic agent that provides therapeutic benefits in treating the condition, alone or in combination with other therapies. The term "therapeutically effective amount" may include an amount that improves the overall treatment, reduces or avoids symptoms, signs or causes, and/or enhances the therapeutic efficacy of another therapeutic agent.

The pharmaceutical composition described herein can be prepared by any method known in the field of pharmacology. Generally, this preparation method comprises combining the compound described herein (i.e., the "active ingredient") with a carrier or excipient, and/or bringing the compound into contact with one or more other auxiliary agents, and then, if necessary and/or needed, molding the product and/or packaging the product into the desired single- or multidose unit.

The pharmaceutical composition can be prepared, packaged and/or sold as a whole batch, as a single unit dose and/or a plurality of single unit doses. The relative amounts of the active ingredient, pharmaceutically acceptable excipients and/or any additional ingredients in the pharmaceutical composition described herein will vary depending on the identity, size and/or the condition of the subject to be treated, as well as the route of the composition to be administered. The composition may comprise 0.1% to 100% (w/w) of the active ingredient.

Pharmaceutically acceptable excipients used in the preparation of the provided pharmaceutical composition include an inert diluent, a dispersant, and/or a granulating agent, a surfactant and/or an emulsifier, a disintegrant, a binder, a preservative, a buffer, a lubricant and/or an oil. Excipients such as cocoa butter and suppository wax, colorants, coating agents, sweeteners, condiments and spices may also be present in the composition.

Liquid dosage forms for oral and parenteral administrations include pharmaceutically acceptable emulsions, microemulsions, solutions, suspensions, syrups, and elixirs. In addition to the active ingredients, liquid dosage forms may comprise inert diluents commonly used in the art, e.g., water or other solvents, solubilizers and emulsifiers, such as ethanol, isopropanol, ethyl carbonate, ethyl acetate, benzyl alcohol, benzyl benzoate, propylene glycol, 1,3-butanediol, dimethylformamide, oils (e.g., cottonseed oil, peanut oil, corn oil, germ oil, olive oil, castor oil, and sesame oil), glycerol, tetrahydrofurfuryl alcohol, propylene glycol, and sorbitan fatty acid esters, and mixtures thereof. In addition to the inert diluents, oral compositions may comprise adjuvants such as wetting agents, emulsifying and suspending agents, sweeteners, condiments, and spices. In certain embodiments of parenteral administration, the conjugates described herein are mixed with solubilizers (such as alcohols, oils, modified oils, glycols, polysorbates, cyclodextrins, polymers and mixtures thereof).

Injectable dosage forms, for example, sterile injectable aqueous or oily suspensions, can be formulated according to known techniques using suitable dispersants or wetting agents and suspending agents. Sterile injectable dosage forms can be sterile injectable solutions, suspensions or emulsions in nontoxic parenterally acceptable diluents or solvents, for example, 1,3-butanediol solutions. Acceptable carriers and solvents that can be used are water, Ringer's solution, U.S.P., and physiological salt solutions. In addition, sterile fixed oils are usually used as solvents or suspending media. For this purpose, any mild fixed oils that can be used include synthetic monoglycerides or diglycerides. In addition, fatty acids (such as oleic acid) are used for preparing the injectable dosage forms.

In order to prolong the effect of a drug, it is usually desirable to reduce the absorption from subcutaneous injection or intramuscular injection. This can be achieved by using a liquid suspension of crystals or amorphous materials with poor water solubility. The absorption rate of the drug depends on the dissolution rate, which in turn depends on the crystal size and crystal form. Alternatively, delayed absorption of a drug form for parenteral administration can be achieved by dissolving or suspending the drug in an oily carrier.

Solid dosage forms for oral administration include capsules, tablets, pills, powders, and granules. In such a solid dosage form, the active ingredient is mixed with at least one of the following inert, pharmaceutically acceptable substances: excipients or carriers, such as sodium citrate or dicalcium phosphate, and/or (a) fillers or bulking agents, such as starch, lactose, sucrose, glucose, mannitol, and silicic acid, (b) binders, such as, for example, carboxymethyl cellulose, alginate, gelatin, polyvinylpyrrolidone, sucrose, and arabic gum, (c) humectants, such as glycerol, (d) disintegrants, such as agar, calcium carbonate, potato or tapioca starch, alginic acid, certain silicates, and sodium carbonate, (e) solution anticoagulants, such as vaseline, (f) absorption enhancers such as quaternary ammonium compounds, (g) wetting agents, such as, for example, cetyl alcohol and glycerol monostearate, (h) absorbents, such as kaolin and bentonite, and (i) lubricants, such as talc, calcium stearate, magnesium stearate, solid polyethylene glycol, sodium dodecyl sulfate, and mixtures thereof. In the case of capsules, tablets, or pills, the dosage form may comprise a buffer.

The active ingredient may be in the form of microcapsules having one or more of the excipients described above. Tablets, dragees, capsules, pills, and granules in the form of solid dosage forms can be prepared by using coatings and shells (such as enteric coatings, release control agent coatings and other coatings known in the field of pharmaceutical preparations). In such a solid preparation, the active ingredient can be mixed with at least one inert diluent (such as sucrose, lactose or starch). Conventionally, such a dosage form may comprise other substances other than inert diluents, for example, tabletting lubricants and other tabletting auxiliary agents such as magnesium stearate and microcrystalline cellulose. In the case of capsules, tablets, or pills, the dosage form may comprise a buffer. They may optionally comprise an opacifying agent and may be compositions having the following properties: they release the active ingredient only, or preferably, in certain parts of the intestinal tract, optionally in a delayed manner. Examples of encapsulating agents that can be used include polymers and waxes.

Although the description of the pharmaceutical composition provided herein is mainly directed at pharmaceutical compositions suitable for administration to humans, such compositions are generally suitable for administration to all types of animals. Modification of the pharmaceutical composition suitable for administration to humans to make the composition suitable for administration to a variety of animals is easy to understand, and those skilled in the art can design and/or use routine experiments for such modifications.

The compounds provided herein are usually formulated in the form of dosage units to facilitate administration and dosage uniformity. However, it will be understood that the daily use of the composition described herein in all instances will be determined by a physician within reasonable medical judgment. The specific therapeutically effective dose level for any particular subject or organism depends on a series of factors, including: the severity of the disease and symptoms to be treated; the activity of the specific active ingredient used; the specific composition used; the age, weight, health, sex, and diet of the subject; the time of administration, route of administration, and excretion rate of the specific active ingredient; the duration of treatment; a drug in combination with or consistent with the specific active ingredient used; and other factors known in the medical field.

In addition, the present disclosure also covers a kit (such as a pharmaceutical package). The kit provided can comprise the pharmaceutical composition or the compound described herein and a container (e.g., drug bottle, ampoule, bottle, syringe and/or sub-package or other suitable container). In some embodiments, the kit provided may optionally further comprise a second container containing a pharmaceutical excipient for diluting or suspending the pharmaceutical composition or the compound described herein. In some embodiments, the pharmaceutical compositions or the compounds described herein disposed in the first container and the second container are combined to form a unit dosage form.

The compounds and the compositions provided herein can be administered by conventional routes, including enteral (e.g., oral) administration, parenteral administration, intravenous administration, intramuscular administration, intra-arterial administration, intramedullary administration, intracapsular administration, subcutaneous administration, intraventricular administration, percutaneous administration, subcutaneous administration, rectal administration, intravaginal administration, intraperitoneal administration, and local administration (e.g., by powders, ointments, creams and/or drops). Particularly contemplated routes are oral administration, intravenous administration (for example, systemic intravenous injection), local administration through blood and/or lymphatic supply and/or direct administration to a predetermined site. Generally, the most suitable route of administration will depend on a series of factors, including: the nature of the agent (e.g., the stability in the gastrointestinal environment) and/or the condition of the subject (e.g., whether oral administration can be tolerated).

The exact amount of the compound required to achieve an effective amount will vary from subject to subject, depending on, for example, the race, age and general condition of the subject, the severity of side effects or condition, the identification of the specific compound, the mode of administration, etc. An effective amount may be included in a single dose (e.g., a single oral dose) or multiple doses (e.g., multiple oral doses). In certain embodiments, when multiple doses are administered to a subject or applied to a biological sample, tissue or cell, any two doses of the multiple doses comprise the different compounds described herein or substantially the same compound described herein. In certain embodiments, when multiple doses are administered to a subject or applied to a biological sample, tissue or cell, the frequency of administration of the multiple doses to a subject or application of multiple doses to a tissue or cell is three doses per day, two doses per day, one dose per day, one dose every two days, one dose every three days, or one dose per week. In certain embodiments, the frequency of administration of multiple doses to a subject or application of multiple doses to a tissue or cell is one dose per day. In certain embodiments, the frequency of administration of multiple doses to a subject or application of multiple doses to a tissue or cell is two doses per day. In certain embodiments, when multiple doses are administered to a subject or applied to a biological sample, tissue or cell, the duration between the first dose and the last dose of the multiple doses is one day, two days, four days, one week, two weeks, three weeks, one month, two months, three months, four months, six months, nine months, one year, two years, three years, four years, five years, seven years, ten years, fifteen years, twenty years, or the life span of the subject, biological sample, tissue or cell. In certain embodiments, the duration between the first dose and the last dose of the multiple doses is three months, six months, or one year. In certain embodiments, the duration between the first dose and the last dose of the multiple doses is the life span of the subject, biological sample, tissue or cell. In certain embodiments, the dosage described herein (e.g., any dosage of a single dose or multiple doses) independently comprises 1 mg to 3 mg, 3 mg to 10 mg, 10 mg to 30 mg, 30 mg to 100 mg, 100 mg to 300 mg, 300 mg to 1,000 mg, or 1 g to 10 g of the compound described herein. In certain embodiments, the dosage described herein independently comprises 3 mg to 10 mg of the compound described herein. In certain embodiments, the dosage described herein independently comprises 10 mg to 30 mg of the compound described herein. In certain embodiments, the dosage described herein independently comprises 30 mg to 100 mg of the compound described herein. In certain embodiments, the dosage described herein independently comprises 100 mg to 300 mg of the compound described herein. In certain embodiments, the dosage described herein independently comprises 300 mg to 1000 mg of the compound described herein.

The term "cancer" refers to a class of diseases characterized by abnormal development of cells, wherein the abnormal cells proliferate uncontrollably and have the ability to infiltrate and destroy normal body tissues. See, for example, Stedman's Medical Dictionary, 25th ed.; Hensyl ed.; Williams & Wilkins: Philadelphia, 1990.

The following examples are only listed as examples of the embodiments of the present disclosure and do not constitute any restrictions on the present disclosure. Those skilled in the art can understand that modifications without departing from the essence and concept of the present disclosure all fall within the scope of protection of the present disclosure. Unless otherwise specified, the reagents and the instruments used in the following examples are all commercially available products.

¹H NMR spectrum is measured by Bruker instrument (400 MHz), and chemical shift is expressed in ppm. An internal standard of tetramethylsilane (0.00 ppm) is used. Representation method for ¹H NMR: s = singlet, d = doublet, t = triplet, m = multiplet, br = broad, dd = doublet of doublets, and dt = doublet of triplets. If a coupling constant is provided, the unit thereof is Hz.

Mass spectrum is obtained by measurement with an LC/MS instrument, and the ionization mode may be ESI or APCI.

Yantai Huanghai HSGF254 or Qingdao GF254 silica gel plates are used as silica gel plates for thin-layer chromatography, the specifications of silica gel plates used for thin-layer chromatography (TLC) are 0.15-0.2 mm, and the specifications used for the separation and purification of products by thin-layer chromatography are 0.4-0.5 mm.

For column chromatography, Yantai Huanghai silica gel 200-300 mesh silica gel is generally used as a carrier.

In the following examples, unless otherwise specified, all temperatures are in degree Celsius. Unless otherwise specified, various starting materials and reagents are commercially available or synthesized according to known methods, and the commercially available raw materials and reagents are directly used without further purification. Unless otherwise specified, manufacturers of commercially available products include, but are not limited to, Aldrich Chemical Company, ABCR GmbH & Co. KG, Acros Organics, Guangzan Huagong Keji Youxian Gongsi, Jingyan Huagong Keji Youxian Gongsi, etc.
CD₃OD: deuterated methanol.
CDCl₃: deuterated chloroform.
DMSO-*d*₆: deuterated dimethyl sulfoxide.
D₂O: heavy water.

Argon atmosphere means that the reaction flask is connected to an argon balloon with a volume of about 1 L.

Unless otherwise specified in the examples, a solution in a reaction refers to an aqueous solution.

For the purification of a compound, C18 reversed-phase column preparative or semi-preparative purification, a silica gel column chromatography eluent system, and thin-layer chromatography are used, wherein the eluent system is selected from: A: petroleum ether and tetrahydrofuran system; B: acetonitrile and water system; and C: petroleum ether and ethyl acetate system; wherein the volume ratio of the solvents varies depending on the polarity of the compound, and adjustment may also be carried out by adding a small amount of acidic or alkaline reagents, such as trifluoroacetic acid, acetic acid or triethylamine.

### Synthesis of intermediate

### Synthesis of 4-fluoro-1,2-dichlorobenzyl (intermediate 1):

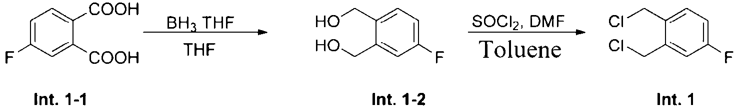

**step 1:** At 0°C, BH₃·THF (6.52 mL, 6.52 mmol, 1M) was added to the compound **4-fluorophthalic acid** (400 mg, 2.17 mmol) in THF (14.5 mL) and stirred at 25°C for 2 h. The reaction mixture was quenched by adding MeOH (30 mL), spin-dried, and purified by silica gel column chromatography (DCM : MeOH = 10 : 1) to obtain the compound **4-fluoro-1,2-benzenedimethanol** (404.00 mg, crude) as a colorless oil.

### LC-MS [M-17]⁺ = 139.2

**step 2:** The compound **4-fluoro-1,2-benzenedimethanol** (236 mg, 1.51 mmol), SOCl₂ (899.01 mg, 7.56 mmol), and DMF (220.94 mg, 3.02 mmol) were dissolved in toluene (15 mL) and stirred at 60°C for 2 h. The reaction mixture was spin-dried. After a saturated sodium chloride aqueous solution (30 mL) was added, the mixture was extracted with ethyl acetate (30 mL × 3), the organic phases were combined, spin-dried, and purified by silica gel column chromatography (PE : THF = 20 : 1) to obtain **intermediate 1** compound (167.00 mg, yield: 57.14%) as a colorless liquid.

¹H-NMR (400MHz, CDCl₃) δ7.39-7.35 (m, 1H), 7.14 (dd, *J* = 8.8,2.4Hz, 1H), 7.06-7.01 (m, 1H), 4.71 (s, 4H).

### Synthesis of 4-trifluoromethyl 1,2-dichlorobenzyl (intermediate 2):

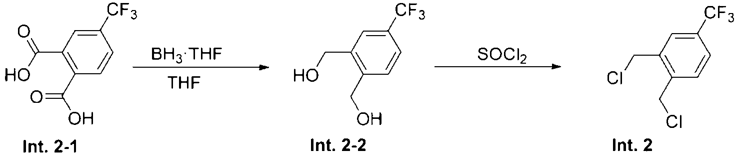

**step 1: 4-Trifluoromethylphthalic acid** (770 mg, 3.29 mol) was dissolved in THF (33 mL). At 0°C, BH₃·THF (1M, 9.87 mL) was added, and the mixture was stirred for 3 h at 20°C. At 0°C, MeOH (23 mL) was then dropwise added for quenching, and the quenched product was concentrated and then purified by silica gel column chromatography (DCM : MeOH = 20 : 1) to obtain **4-trifluoromethyl 1,2-benzenedimethanol** (820.00 mg, crude) as a colorless oil.

### LC-MS [M-17]⁺ = 189.1

**step 2: 4-Trifluoromethyl 1,2-benzenedimethanol** (820 mg, 3.93 mol) and DMF (574.36 mg, 7.86 mmol, 608.44 uL) were dissolved in toluene (36 mL) and stirred for 2 h at 60°C under nitrogen protection. After concentration, the concentrated product was purified by silica gel column chromatography (PE : THF = 20 : 1) to obtain **intermediate 2** (410.00 mg, yield: 39.8%) as a colorless liquid.

¹H NMR (400MHz, CDCl₃) δ7.61 (s, 1H), 7.60 (dd, *J* = 8.0,1.2Hz, 1H), 7.53 (d, *J =* 8.0 Hz, 1H), 4.76-4.74 (m, 4H).

### Example 1: Synthesis of 6-(isoindolin-2-ylmethyl)-1-methyl-3-((1-(methylsulfonyl) piperidine-4-yl)methoxy)pyridin-2(1H)-one:

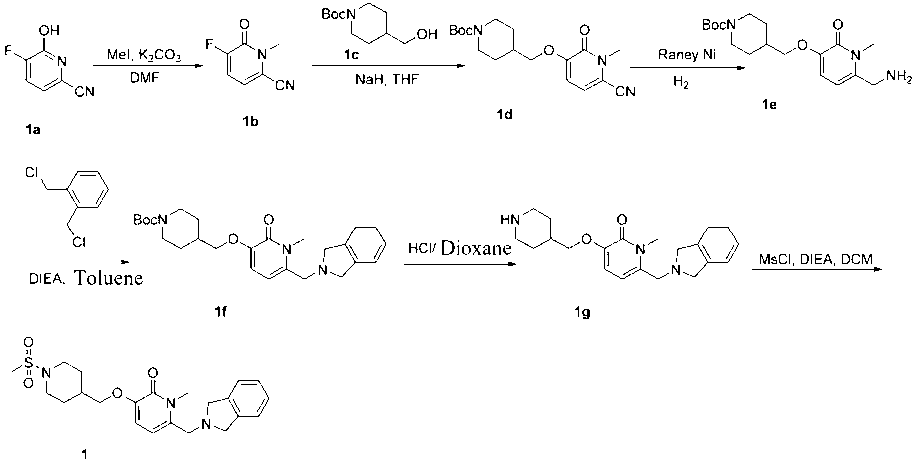

### step 1: Synthesis of 5-fluoro-1-methyl-6-oxo-1,6-dihydropyridine-2-carbonitrile (1b):

The compound 5-fluoro-6-hydroxycyanopyridine **1a** (500 mg, 3.62 mmol) was dissolved in DMF (10 mL), and MeI (591.24 mg, 4.16 mmol) and K₂CO₃ (999.28 mg, 7.24 mmol) were then separately added. At 25°C, the mixture was stirred for 1 hour. After saturated NaCl (30 mL) was added to the reaction mixture, the reaction mixture was extracted with ethyl acetate (20 mL × 3), and the organic phases were combined, dried, spin-dried, and purified by silica gel column chromatography (PE : THF = 3 : 1) to obtain compound **1b** (312.00 mg, yield: 56.65%) as a white solid.
LC-MS [M+1]⁺ = 153.1
¹H-NMR (400MHz, DMSO) δ7.53 (dd, *J* = 9.6,8.0Hz, 1H), 7.18 (dd, *J* = 7.6,4.8Hz, 1H), 3.60 (s, 3H).

### step 2: Synthesis of tert-butyl 4-(((6-cyano-1-methyl-2-oxo-1,2-dihydropyridin-3-yl)oxy)methyl)piperidine-1-carbonate 1d:

At 0°C, the compound tert-butyl-4-(hydroxymethyl)piperidine-1-carbonate **1c** (2.21g, 10.25 mmol) was dissolved in THF (20 mL), NaH (656.24 mg, 16.41 mmol, 60% purity) was slowly added, and the mixture was stirred for 30 minutes under nitrogen protection. At 0°C, the compound **1b** (312 mg, 2.05 mmol) was added to the reaction mixture, and the mixture was heated to 25°C and stirred for 1 hour under nitrogen protection. The reaction mixture was quenched by pouring into ice water and extracted with ethyl acetate (50 mL × 3) and saturated NaCl (30 mL × 3), and the organic phases were combined, dried, spin-dried, and purified by silica gel column chromatography [PE (0.1% NH₄ OH) : THF = 0 : 1] to obtain compound **1d** (602.00 mg, yield: 80.01%) as a white solid.
LC-MS [M+1]⁺ = 348.1

### step 3: Synthesis of tert-butyl 4-(((6-(aminomethyl)-1-methyl-2-oxo-1,2-dihydropyridin-3-yl)oxy)methyl)piperidine-1-carbonate 1e:

Compound **1d** (257 mg, 0.74 mmol) and Raney nickel (43.42 mg, 0.74 mmol) were dissolved in a mixed solution of methanol (10 mL), tetrahydrofuran (10 mL), and aqueous ammonia (2 mL), and the mixture was stirred for 2 hours at 25°C under hydrogen condition. The reaction mixture was filtered with methanol (50 mL × 3), and the organic phases were combined, dried, spin-dried to obtain compound **1e** (258.00 mg, crude) as a yellow oil.
LC-MS [M+1]⁺=352.2

### step 4: Synthesis of tert-butyl 4-(((6-(isoindolin-2-ylmethyl)-1-methyl-2-oxo-1,2-dihydropyridin-3-yl)oxy)methyl)piperidine-1-carbonate 1f:

Compound **1e** (100 mg, 284.54 µmol) was dissolved in toluene (3 mL), and the compound o-dichlorobenzyl (74.72 mg, 0.43 mmol) and DIEA (110.12 mg, 0.85mmol) were subsequently added and stirred for 2 hours at 100°C. The reaction mixture was spin-dried and extracted with ethyl acetate (30 mL × 3) and H₂O (10 mL × 3), and the organic phases were combined, dried, spin-dried. After purification by silica gel column chromatography (PE : THF = 0 : 1), compound **1f** (49.00 mg, yield: 32.48%) was obtained as a yellow oil.
LC-MS [M+1]⁺ = 454.4

### step 5: Synthesis of 6-(isoindolin-2-ylmethyl)-1-methyl-3-(piperidine-4-ylmethoxy)pyridin-2(1H)-one 1g:

Compound **1e** (46 mg, 101.42 umol) was dissolved in HCl/dioxane (4M, 20 mL) and stirred for 30 minutes at 25°C. The reaction mixture was directly spin-dried to obtain compound 1g (75.00 mg, crude) as a gray solid. The product could be directly used in the next reaction without purification.

### step 6: Synthesis of 6-(isoindolin-2-ylmethyl)-1-methyl-3-((1-(methylsulfonyl)piperidine-4-yl)methoxy)pyridin-2(1H)-one (compound 1):

Compound **1g** (75 mg, 192.35 umol) and DIEA (74.44 mg, 577.04 umol) were mixed and dissolved in dichloromethane (2 mL), and at 0°C, MsCl (26.44 mg, 230.82 umol) was slowly added. At 0°C, the mixture was stirred for 30 minutes. The reaction mixture was directly concentrated and purified by Prep. HPLC [ACN : H₂O (0.1% NH₄HCO₃)] to obtain compound **1** (5.84 mg, yield: 7.04%) as a white solid. (Free base)

LC-MS [M+1]⁺ = 432.1
¹H-NMR (400MHz, MeOD) δ7.20-7.16 (m, 4H), 6.89 (d, *J =* 8.0 Hz, 1H), 6.38 (d, *J =* 7.6H_{Z}, 1H), 3.91 (s, 4H), 3.87 (s, 2H), 3.84 (d, *J =* 6.0 Hz, 2H), 3.78-3.74 (m, 5H), 2.83-2.75 (m, 5H), 2.06-2.04 (m, 1H), 2.02-1.97 (m, 2H), 1.48-1.38 (m, 2H).

### Example 2: Synthesis of 6-(isoindolin-2-ylmethyl)-3-((1-(methylsulfonyl)piperidine-4-yl)methoxy)pyridin-2(1H)-one

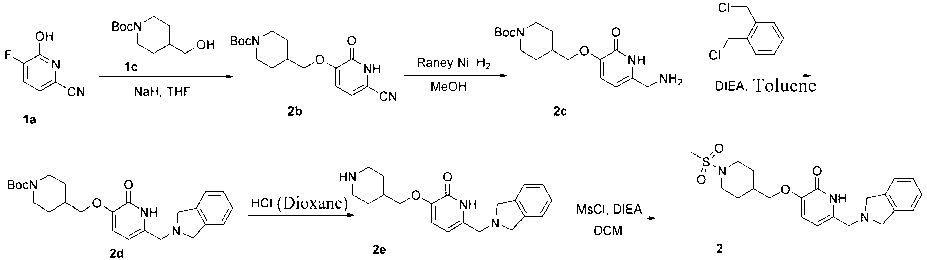

### step 1: Synthesis of tert-butyl 4-(((6-cyano-2-oxo-1,2-dihydropyridin-3-yl)oxy)methyl)piperidine-1-carbonate (2b):

Compound **1a** (300 mg, 2.17 mmol) was dissolved in THF (22 mL), NaH (695.15 mg, 17.38mmol) was added at 0°C, and the mixture was stirred for 30 minutes. Subsequently, **1c** was then added to the reaction mixture, and the reaction was heated to room temperature and further stirred for 1 hour. The reaction mixture was quenched with saturated NaHCO₃ (20 mL). After extraction with EA (20 mL x 3), the organic phases were combined. After purification by silica gel column chromatography (PE : THF = 3.3 : 1), the desired compound **2b** (153 mg, yield: 15.00%) was obtained as a light yellow oil.
LC-MS [M-56]⁺ = 278.1

### step 2: Synthesis of tert-butyl 4-(((6-(aminomethyl)-2-oxo-1,2-dihydropyridin-3-yl)oxy)methyl)piperidine-1-carbonate (2c):

Compound **2b** (153 mg, 2.18 mmol) was dissolved in MeOH (15 mL), and Raney nickel (26.93 mg, 458.93 umol) was added. After continued stirring for 1 hour at 25°C in a hydrogen atmosphere, filtration was carried out, and the filtrate was directly spin-dried to obtain crude compound 2c (153.00 mg, crude) as a brown oil, which could be directly used in the next reaction without purification.
LC-MS [M+1]⁺ = 338.3

### step 3: Synthesis of tert-butyl 4-(((6-(isoindolin-2-ylmethyl)-2-oxo-1,2-dihydropyridin-3-yl)oxy)methyl)piperidine-1-carbonate (2d):

Compound **2c** (153 mg, 136.04 umol) and the compound o-dichlorobenzyl (19.05 mg, 108.83 umol) and DIEA (52.74 mg, 408.11 umol) were dissolved in toluene (2 mL) and stirred for 16 hours at 100°C. The reaction mixture was directly spin-dried and purified by Prep-TLC [PE (0.1% NH₄OH) : THF = 1 : 4] to obtain compound **2d** (50.00 mg, yield: 26.45%) as a brown solid.
LC-MS [M+1]⁺ = 440.3

### step 4: Synthesis of 6-(isoindolin-2-ylmethyl)-3-(piperidine-4-ylmethoxy)pyridin-2(1H)-one (2e):

Compound **2d** (50 mg, 113.75 umol) was dissolved in HCl/dioxane (4 mL) and stirred for 30 minutes at 25°C. The reaction solution was directly removed under reduced pressure to obtain compound **2e** (50.00 mg, crude) as a brown solid. The product could be directly used in the next reaction without purification.
LC-MS [M+1]⁺ = 340.2

### step 5: Synthesis of 6-(isoindolin-2-ylmethyl)-3-((1-(methylsulfonyl)piperidine-4-yl)methoxy)pyridin-2(1H)-one (2)

Compound **2e** (50 mg, 146.44 umol) and DIEA (56.78 mg, 439.31 umol) were mixed and dissolved in DCM (4 mL), and at 0°C, MsCl (20.13 mg, 175.72 umol) was slowly added. At 0°C, the mixture was stirred for 30 minutes. The reaction solution was directly removed under reduced pressure to obtain a brown oil. The mixture was dissolved in THF (2.2 mL) and H₂O (1.4 mL), and subsequently, LiOH (4.28 mg, 178.77 umol) was added. At 25°C, the mixture was stirred for 1 hour. The reaction mixture was purified by Prep. HPLC [ACN : H₂O (0.1% NH₄HCO₃)] to obtain **compound 2** (10.93 mg, purity: 98.07%, yield: 43.58%) as a white solid.

LC-MS [M-1]- = 416.1
¹HNMR (400MHz, DMSO-d6) δ11.43 (s, 1H), 7.20-7.13 (m, 4H), 6.75 (d, *J =* 8.0H_{Z}, 1H), 6.04 (d, *J* = 8.0H_{Z}, 1H), 3.81 (s, 4H), 3.72 (d, *J* = 6.0H_{Z}, 2H), 3.58-3.54 (m, 4H), 2.82 (s, 3H), 2.72-2.67 (m, 2H), 1.84-1.81 (m, 3H), 1.32-1.24 (m, 2H).

### Example 3. Synthesis of 3-((1-(cyclopropylsulfonyl)piperidine-4-yl)methoxy)-6-(isoindolin-2-ylmethyl)-1-methylpyridin-2(1H)-one (compound 3):

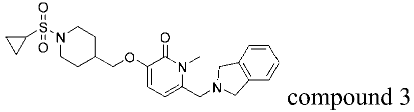

Similarly to the synthesis route of Example **1,** the above compound could be obtained by replacing the methylsulfonyl chloride in step 6 with cyclopropylsulfonyl chloride.

LC-MS [M+1]⁺ = 458.1
¹HNMR (400MHz, DMSO-d6) δ7.24-7.17 (m, 4H), 6.79 (d, *J* = 7.6H_{Z}, 1H), 6.20 (d, *J* = 7.6H_{Z}, 1H), 3.86 (s, 4H), 3.80-3.77 (m, 4H), 3.66-3.62 (m, 2H), 3.56 (s, 3H), 2.89-2.82 (m, 2H), 2.53-2.50 (m, 1H), 1.96-1.84 (m, 3H), 1.37-1.27 (m, 2H), 1.01 (m, 2H), 0.90 (m, 2H).

### Example 4. Synthesis of 4-(((6-(isoindolin-2-ylmethyl)-1-methyl-2-oxo-1,2-dihydropyridin-3-yl)oxy)methyl)-N,N-dimethylpiperidine-1-sulfonamide (compound 4)

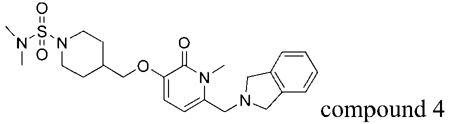

Similarly to the synthesis route of Example **1,** the above compound could be obtained by replacing the methylsulfonyl chloride in step 6 with dimethylaminosulfonyl chloride.

LC-MS [M+1]⁺ = 460.9
¹HNMR (400MHz, DMSO-d6) δ7.24-7.17 (m, 4H), 6.78 (d, *J* = 7.6H_{Z}, 1H), 6.20 (d, *J* = 7.6H_{Z}, 1H), 3.86 (s, 4H), 3.80 (s, 2H), 3.76 (d, *J* = 6.4Hz, 2H), 3.60 (d, *J* = 12.4Hz, 2H), 3.55 (s, 3H), 2.89-2.82 (m, 2H), 2.75 (s, 6H), 1.80 (m, 3H), 1.32-1.22 (m, 2H).

### Example 5. Synthesis of 6-((5-fluoroisoindolin-2-yl)methyl)-1-methyl-3-((1-(methylsulfonyl)piperidine-4-yl)methoxy)pyridin-2(1H)-one (compound 5):

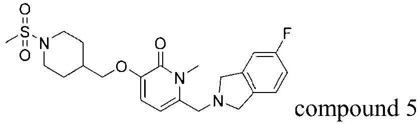

Similarly to the synthesis route of Example **1,** the above compound could be obtained by replacing the o-dichlorobenzyl in step 4 with 4-fluoro-1,2-dichlorobenzyl (intermediate 1).

LC-MS [M+1]⁺ = 450.1
¹HNMR (400MHz, DMSO-d6) δ7.26-7.2 (m, 1H), 7.10-7.07 (m, 1H), 7.02-6.99 (m, 1H), 6.79 (d, *J =* 7.6H_{Z}, 1H), 6.20 (d, *J* = 7.6H_{Z}, 1H), 3.85-3.76 (m, 8H), 3.60-3.57 (m, 2H), 3.55 (s, 3H), 2.86 (s, 3H), 2.77-2.70 (m, 2H), 1.86 (d, *J =* 10 Hz, 3H), 1.37-1.28 (m, 2H).

### Example 6. Synthesis of 6-(isoindolin-2-ylmethyl)-1-methyl-3-((1-(oxetan-3-ylmethylsulfonyl)piperidine-4-yl)methoxy)pyridin-2(1H)-one (compound 6):

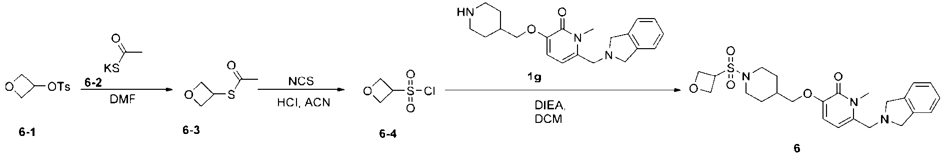

**step 1: 6-1** (1 g, 4.38 mmol) and potassium thioacetate **6-2** (1.00 g, 8.76 mmol) were dissolved in DMF (20 mL) and stirred for 4 h at 100°C. After concentration to remove DMF, water (20 mL) and ethyl acetate (30 mL) were added, and liquid separation was carried out. After the aqueous phase was extracted three times with ethyl acetate (30 mL), the organic phases were combined, dried, concentrated, and then purified by silica gel column chromatography (PE : THF = 92 : 8) to obtain **6-3** (167.00 mg, yield: 25.96%) as a colorless oil.
LC-MS [M-56]⁺ = 132.9

**step 2: 6-3** (160 mg, 1.21 mmol) and a hydrochloric acid aqueous solution (2M, 151.31 µL) were dissolved in acetonitrile (4.85 mL), and after NCS (646.55 mg, 4.84 mmol) was added in portions at 0°C, the mixture was stirred for 1 h at 0°C, then concentrated, and then purified by silica gel column chromatography (PE : THF = 85 : 15) to obtain **6-4** (130.00 mg, yield: 61.73%) as a colorless oil.

¹HNMR (400MHz, CDCl₃) δ5.29-4.93 (m, 5H).

**step 3: 1 g** (30 mg, 42.44 µmol) and DIEA (16.45 mg, 127.31 µmol) were dissolved in DCM (2 mL), and after **6-4** (6.65 mg, 42.44 µmol) was added at 0°C, the mixture was then stirred for 30 min at 0°C, then concentrated, and then purified by Prep. HPLC [ACN : H₂O (0.1% NH₄HCO₃)] to obtain **6** (8.28 mg, yield: 40.84%, purity: 99.12%) as a white solid.
LC-MS [M+1]⁺ = 474.3

¹HNMR (400MHz, DMSO-d6) δ7.20-7.14 (m, 4H), 6.75 (d, *J =* 7.6H_{Z}, 1H), 6.17 (d, *J =* 7.6H_{Z}, 1H), 4.81-4.66 (m, 5H), 3.82 (s, 4H), 3.76 (s, 2H), 3.73-3.72 (m, 2H), 3.61-3.57 (m, 2H), 3.52 (s, 3H), 2.79-2.73 (m, 2H), 1.88-1.76 (m, 3H), 1.24-1.16 (m, 2H).

### Example 7. Synthesis of 3-((1-acetylpiperidine-4-yl)methoxy)-6-(isoindolin-2-ylmethyl)-1-methylpyridin-2(1H)-one (compound 7):

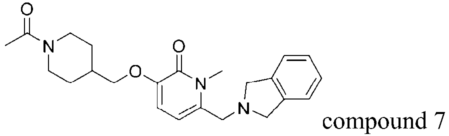

Similarly to the synthesis route of Example **1,** the above compound could be obtained by replacing the methylsulfonyl chloride in step 6 with acetyl chloride.

LC-MS [M+1]⁺ = 396.1
¹HNMR (400MHz, DMSO-*d6*) δ7.24-7.17 (m, 4H), 6.78 (d, *J* = 7.6H_{Z}, 1H), 6.20 (d, *J* = 7.6H_{Z}, 1H), 4.39 (d, *J =* 13.2Hz, 1H), 3.86-3.74 (m, 9H), 3.55 (s, 3H), 3.08-3.01 (m, 1H), 2.52-2.49 (m, 2H), 1.99 (s, 3H), 1.83-1.72 (m, 2H), 1.27 (m, 1H), 1.03 (m, 1H).

### Example 8. Synthesis of 3-((1-acetylpiperidine-4-yl)methoxy)-6-(isoindolin-2-ylmethyl)-1-methylpyridin-2(1H)-one (compound 8):

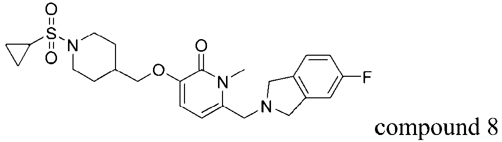

Similarly to the synthesis route of Example **1,** the above compound could be obtained by replacing the o-dichlorobenzyl in step 4 with 4-fluoro-1,2-dichlorobenzyl (intermediate 1) and replacing the methylsulfonyl chloride in step 6 with cyclopropylsulfonyl chloride.

LC-MS [M+1]⁺ = 476.1
¹HNMR (400MHz, DMSO-d6) δ7.22-7.20 (m, 1H), 7.10-7.07 (m, 1H), 7.02-6.98 (m, 1H), 6.78 (d, *J =* 8.0H_{Z}, 1H), 6.20 (d, *J =* 7.6H_{Z}, 1H), 3.83 (d, *J =* 11.6Hz, 4H), 3.79-3.76 (m, 4H), 3.64 (d, *J* = 12Hz, 2H), 3.55 (s, 3H), 2.89-2.82 (m, 2H), 2.59-2.54 (m, 1H), 1.90-1.84 (m, 3H), 1.34-1.28 (m, 2H), 1.00-0.96 (m, 2H), 0.93-0.90 (m, 2H).

### Example 9. Synthesis of methyl4-(((6-(isoindolin-2-ylmethyl)-1-methyl-2-oxo-1,2-dihydropyridin-3-yl)oxy)methyl)piperidine-1-carbonate (compound 9):

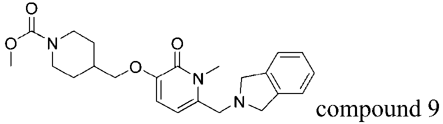

Similarly to the synthesis route of Example **1,** the above compound could be obtained by replacing the methylsulfonyl chloride in step 6 with methyl chloroformate.

LC-MS [M+1]⁺ = 411.6
¹HNMR (400MHz, DMSO-*d6*) δ7.24-7.17 (m, 4H), 6.78 (d, *J* = 7.6H_{Z}, 1H), 6.20 (d, *J* = 7.6H_{Z}, 1H), 4.05 (m, 2H), 3.85 (s, 4H), 3.79 (m, 2H), 3.74 (d, *J =* 6.0 Hz, 2H), 3.59 (s, 3H), 3.55 (s, 3H), 2.82 (m, 2H), 2.00 (m, 1H), 1.75 (d, *J =* 15.2Hz, 2H), 1.21-1.11 (m, 2H).

### Example 10. Synthesis of 6-(isoindolin-2-ylmethyl)-1-methyl-3-((1-((trifluoromethyl)sulfonyl)piperidine-4-yl)methoxy)pyridin-2(1H)-one (compound 10):

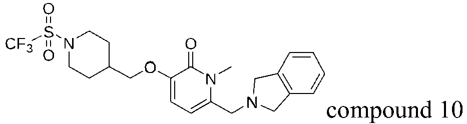

Similarly to the synthesis route of Example **1,** the above compound could be obtained by replacing the methylsulfonyl chloride in step 6 with trifluoromethanesulfonic anhydride.

LC-MS [M+1]⁺ = 486.1
¹HNMR (400MHz, DMSO-d6) δ7.23-7.17 (m, 4H), 6.79 (d, *J* = 7.6H_{Z}, 1H), 6.21 (d, *J* = 7.6H_{Z}, 1H), 3.85-3.78 (m, 10H), 3.55 (s, 3H), 3.27-3.21 (m, 2H), 2.07 (m, 1H), 1.91 (d, *J =* 12.8Hz, 2H), 1.37-1.27 (m, 2H).

### Example 11. Synthesis of 4-(((6-((5-fluoroisoindolin-2-yl)methyl)-1-methyl-2-oxo-1,2-dihydropyridin-3-yl)oxy)methyl)-N,N-dimethylpiperidine-1-sulfonamide (compound 11):

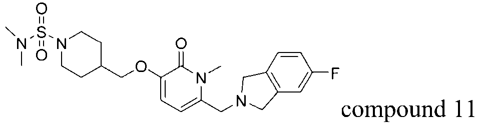

Similarly to the synthesis route of Example **1,** the above compound could be obtained by replacing the o-dichlorobenzyl in step 4 with 4-fluoro-1,2-dichlorobenzyl (intermediate 1) and replacing the methylsulfonyl chloride in step 6 with dimethylaminosulfonyl chloride.

LC-MS [M+1]⁺ = 479.3
¹HNMR (400MHz, DMSO-d6) δ7.26-7.22 (m, 1H), 7.09-7.07 (m, 1H), 7.02-6.98 (m, 1H), 6.78 (d, *J =* 8.0H_{Z}, 1H), 6.20 (d, *J =* 7.6H_{Z}, 1H), 3.85-3.75 (m, 8H), 3.59 (d, *J =* 12.4Hz, 2H), 3.55 (s, 3H), 2.89-2.82 (m, 2H), 2.75 (s, 6H), 1.95 (m, 1H), 1.84-1.80 (m, 2H), 1.32-1.22 (m, 2H).

### Example 12. Synthesis of 6-((5-fluoroisoindolin-2-yl)methyl)-1-methyl-3-((1-(oxetan-3-ylsulfonyl)piperidine-4-yl)methoxy)pyridin-2(1H)-one (compound 12):

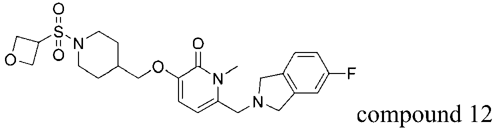

Similarly to the synthesis route of Example **1,** the above compound could be obtained by replacing the o-dichlorobenzyl in step 4 with 4-fluoro-1,2-dichlorobenzyl (intermediate 1) and replacing the methylsulfonyl chloride in step 6 with oxetane-3-sulfonyl chloride (6-4).

LC-MS [M+1]⁺ = 492.1
¹HNMR (400MHz, DMSO-d6) δ7.26-7.22 (m, 1H), 7.10-7.07 (m, 1H), 7.03-6.98 (m, 1H), 6.77 (d, *J =* 8.0H_{Z}, 1H), 6.19 (d, *J =* 7.6H_{Z}, 1H), 4.85-4.82 (m, 2H), 4.78 (m, 1H), 4.72-4.70 (m, 2H), 3.85-3.75 (m, 8H), 3.63 (d, *J =* 12Hz, 2H), 3.55 (s, 3H), 2.83-2.76 (m, 2H), 1.91 (m, 1H), 1.82 (d, *J =* 14.8Hz, 2H), 1.29-1.19 (m, 2H).

### Example 13. Synthesis of 1-methyl-3-((1-(methylsulfonyl)piperidine-4-yl)methoxy)-6-((5-(trifluoromethyl)isoindolin-2-yl)methyl)pyridin-2(1H)-one (compound 13):

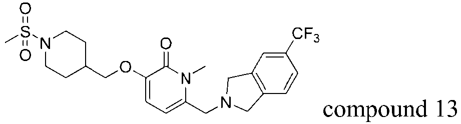

Similarly to the synthesis route of Example **1,** the above compound could be obtained by replacing the o-dichlorobenzyl in step 4 with 4-trifluoromethyl-1,2-dichlorobenzyl (intermediate 2).

LC-MS [M+1]⁺ = 500.1
¹HNMR (400MHz, DMSO-d6) δ7.58 (s, 1H), 7.53 (d, *J =* 8.0 Hz, 1H), 7.42 (d, *J =* 8.0 Hz, 1H), 6.76 (d, *J =* 7.6H_{Z}, 1H), 6.18 (d, *J =* 7.6H_{Z}, 1H), 3.90 (s, 4H), 3.79 (s, 2H), 3.74, (d, *J* = 5.6Hz, 2H), 3.52 (m, 5H), 2.82 (s, 3H), 2.73-2.70 (m, 2H), 1.83 (d, *J =* 10 Hz, 3H), 1.33-1.23 (m, 2H).

### Example 14. Synthesis of 1-(methyl-d3)-3-((1-(methylsulfonyl)piperidine-4-yl)methoxy)-6-((5-(trifluoromethyl)isoindolin-2-yl)methyl)pyridin-2(1H)-one (compound 14):

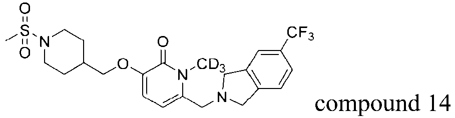

Similarly to the synthesis route of Example **1,** the above compound could be obtained by replacing the iodomethane in step 1 with deuterated iodomethane and replacing the o-dichlorobenzyl in step 4 with 4-trifluoromethyl-1,2-dichlorobenzyl (intermediate 2).

LC-MS [M+1]⁺ = 503.1
¹HNMR (400MHz, DMSO-*d6*) δ7.62 (s, 1H), 7.56 (d, *J =* 8.8Hz, 1H), 7.46 (d, *J =* 7.6Hz, 1H), 6.80 (d, *J =* 7.6H_{Z}, 1H), 6.21 (d, *J* = 7.6H_{Z}, 1H), 3.93 (s, 4H), 3.82 (s, 2H), 3.77, (d, *J* = 6.0 Hz, 2H), 3.59 (d, *J =* 11.6Hz, 2H), 2.86 (s, 3H), 2.76-2.70 (m, 2H), 1.86 (d, *J =* 14.8Hz, 3H), 1.37-1.27 (m, 2H).

### Example 15. Synthesis of 6-((5-fluoroisoindolin-2-yl)methyl)-1-(methyl-d3)-3-((1-(oxetan-3-ylsulfonyl)piperidine-4-yl)methoxy)pyridin-2(1H)-one (compound 15):

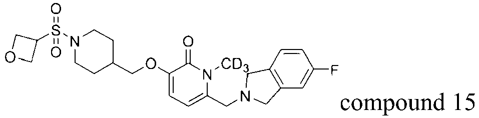

Similarly to the synthesis route of Example **1,** the above compound could be obtained by replacing the iodomethane in step 1 with deuterated iodomethane, replacing the o-dichlorobenzyl in step 4 with 4-fluoro-1,2-dichlorobenzyl (intermediate 1), and replacing the methylsulfonyl chloride in step 6 with oxetane-3-sulfonyl chloride (6-4).

LC-MS [M+1]⁺ = 495.1
¹HNMR (400MHz, DMSO-d6) δ7.22-7.19 (m, 1H), 7.06-7.03 (m, 1H), 6.99-6.94 (m, 1H), 6.74 (d, *J =* 7.6H_{Z}, 1H), 6.16 (d, *J* = 7.6H_{Z}, 1H), 4.81-4.66 (m, 5H), 3.81-3.71 (m, 8H), 3.59 (d, *J* = 12.0 Hz, 2H), 2.79-2.73 (m, 2H), 1.88-1.76 (m, 3H), 1.26-1.17 (m, 2H).

### Example 16. Synthesis of 6-(isoindolin-2-ylmethyl)-1-(methyl-d3)-3-((1-((trifluoromethyl)sulfonyl)piperidine-4-yl)methoxy)pyridin-2(1H)-one (compound 16):

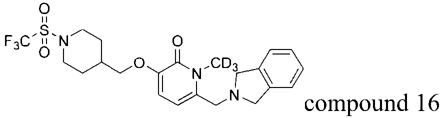

Similarly to the synthesis route of Example **1,** the above compound could be obtained by replacing the iodomethane in step 1 with deuterated iodomethane and replacing the methylsulfonyl chloride in step 6 with trifluoromethanesulfonic anhydride.

LC-MS [M+1]⁺ = 489.1
¹HNMR (400MHz, DMSO-d6) δ7.23-7.17 (m, 4H), 6.79 (d, *J* = 8.0H_{Z}, 1H), 6.21 (d, *J* = 7.6H_{Z}, 1H), 3.85-3.78 (m, 10H), 3.23-3.20 (m, 2H), 2.10 (m, 1H), 1.91 (d, *J =* 13.2Hz, 2H), 1.37-1.27 (m, 2H).

### Example 17. Synthesis of 6-(isoindolin-2-ylmethyl)-1-(methyl-d3)-3-((1-(methylsulfonyl)piperidine-4-yl)methoxy)pyridin-2(1H)-one (compound 17):

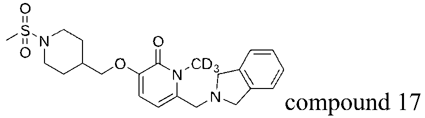

Similarly to the synthesis route of Example **1,** the above compound could be obtained by replacing the iodomethane in step 1 with deuterated iodomethane.

LC-MS [M+1]⁺ = 435.3
¹HNMR (400MHz, DMSO-d6) δ7.20-7.14 (m, 4H), 6.76 (d, *J* = 8.0H_{Z}, 1H), 6.17 (d, *J* = 7.6H_{Z}, 1H), 3.82 (s, 4H), 3.76-3.73 (m, 4H), 3.55 (d, *J =* 11.6Hz, 2H), 2.82 (s, 3H), 2.73-2.67 (m, 2H), 1.83 (d, *J =* 9.6Hz, 3H), 1.32-1.24 (m, 2H).

### Example 18. Synthesis of 3-((1-(cyclopropylsulfonyl)piperidine-4-yl)methoxy)-6-(isoindolin-2-ylmethyl)-1-(methyl-d3)pyridin-2(1H)-one (compound 18):

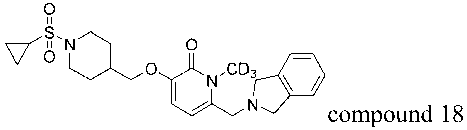

Similarly to the synthesis route of Example **1,** the above compound could be obtained by replacing the iodomethane in step 1 with deuterated iodomethane and replacing the methylsulfonyl chloride in step 6 with cyclopropylsulfonyl chloride.

LC-MS [M+1]⁺ = 460.9
¹HNMR (400MHz, DMSO-*d6*) δ7.20-7.14 (m, 4H), 6.76 (d, *J* = 7.6H_{Z}, 1H), 6.17 (d, *J* = 7.6H_{Z}, 1H), 3.82 (s, 4H), 3.76-3.73 (m, 4H), 3.61 (d, *J =* 12.0 Hz, 2H), 2.85-2.79 (m, 2H), 2.57-2.50 (m, 1H), 1.89-1.80 (m, 3H), 1.33-1.23 (m, 2H), 0.98-0.96 (m, 2H), 0.90-0.86 (m, 2H).

### Example 19. Synthesis of 3-((1-(cyclopropylsulfonyl)piperidine-4-yl)methoxy)-1-(methyl-d3)-6-((5-(trifluoromethyl)isoindolin-2-yl)methyl)pyridin-2(1H)-one (compound 19):

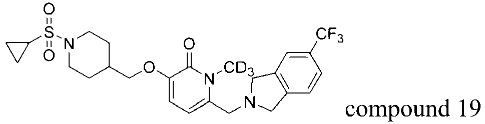

Similarly to the synthesis route of Example **1,** the above compound could be obtained by replacing the iodomethane in step 1 with deuterated iodomethane, replacing the o-dichlorobenzyl in step 4 with 4-trifluoromethyl-1,2-dichlorobenzyl (intermediate 2), and replacing the methylsulfonyl chloride in step 6 with cyclopropylsulfonyl chloride.

LC-MS [M+1]⁺ = 529.2
¹HNMR (400MHz, DMSO-*d6*) δ7.58 (s, 1H), 7.52 (d, *J =* 7.6Hz, 1H), 7.42 (d, *J =* 8.0 Hz, 1H), 6.76 (d, *J =* 7.6H_{Z}, 1H), 6.17 (d, *J =* 7.6H_{Z}, 1H), 3.89 (s, 4H), 3.78 (s, 2H), 3.74 (d, *J =* 6.4Hz, 2H), 3.63-3.59 (m, 2H), 2.85-2.79 (m, 2H), 2.57-2.50 (m, 1H), 1.85-1.80 (m, 3H), 1.30-1.20 (m, 2H), 0.98-0.86 (m, 4H).

### Example 20. Synthesis of 3-((1-(cyclopropylsulfonyl)piperidine-4-yl)methoxy)-6-((5-fluoroisoindolin-2-yl)methyl)-1-(methyl-d3)pyridin-2(1H)-one (compound 20):

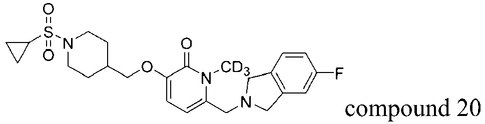

Similarly to the synthesis route of Example **1,** the above compound could be obtained by replacing the iodomethane in step 1 with deuterated iodomethane, replacing the o-dichlorobenzyl in step 4 with 4-fluoro-1,2-dichlorobenzyl (intermediate 1), and replacing the methylsulfonyl chloride in step 6 with cyclopropylsulfonyl chloride.

LC-MS [M+1]⁺= 479.3
¹HNMR (400MHz, DMSO-d6) δ7.22-7.19 (m, 1H), 7.06-7.04 (m, 1H), 6.99-6.94 (m, 1H), 6.75 (d, *J* = 7.6H_{Z}, 1H), 6.16 (d, *J* = 7.6H_{Z}, 1H), 3.81-3.73 (m, 8H), 3.61 (d, *J* = 12.0 Hz, 2H), 2.85-2.79 (m, 2H), 2.56-2.50 (m, 1H), 1.85-1.79 (m, 3H), 1.30-1.20 (m, 2H), 0.96-0.86 (m, 4H).

### Example 21. Synthesis of 3-((1-(cyclopropylsulfonyl)piperidine-4-methyl)methoxy)-1-methyl-6-((5-(trifluoromethyl)isoindolin-2-yl)methyl)pyridin-2(1H)-one (compound 21):

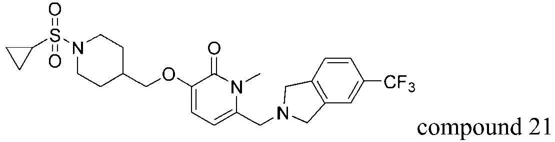

Similarly to the synthesis route of Example **1,** the above compound could be obtained by replacing the o-dichlorobenzyl in step 4 with 4-trifluoromethyl-1,2-dichlorobenzyl (intermediate 2) and replacing the methylsulfonyl chloride in step 6 with cyclopropylsulfonyl chloride.

LC-MS [M+1]⁺ = 526.2
¹HNMR (400MHz, DMSO-d6) δ7.59 (s, 1H), 7.53 (d, *J =* 8.0 Hz, 1H), 7.43 (d, *J =* 8.0 Hz, 1H), 6.76 (d, *J =* 7.6H_{Z}, 1H), 6.18 (d, *J =* 7.6H_{Z}, 1H), 3.90 (s, 4H), 3.79 (s, 2H), 3.74 (d, *J* = 6.4Hz, 2H), 3.61 (d, *J =* 12.4Hz, 2H), 3.51 (s, 3H), 2.85-2.79 (m, 2H), 2.56-2.50 (m, 1H), 1.87-1.81 (m, 3H), 1.30-1.20 (m, 2H), 0.96-0.93 (m, 2H), 0.90-0.86 (m, 2H).

### Example 22. Synthesis of N,N-dimethyl-4-(((1-methyl-2-oxo-6-((5-(trifluoromethyl)isoindolin-2-yl)methyl)-1,2-dihydropyridin-3-yl)oxy)methyl)piperidine-1-sulfonamide (compound 22)

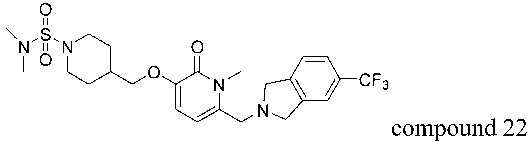

Similarly to the synthesis route of Example **1,** the above compound could be obtained by replacing the o-dichlorobenzyl in step 4 with 4-trifluoromethyl-1,2-dichlorobenzyl (intermediate 2) and replacing the methylsulfonyl chloride in step 6 with dimethylaminosulfonyl chloride.

LC-MS [M+1]⁺ = 529.2
¹HNMR (400MHz, DMSO-d6) δ7.62 (s, 1H), 7.56 (d, *J =* 8.0 Hz, 1H), 7.46 (d, *J =* 8.0 Hz, 1H), 6.79 (d, *J =* 7.6H_{Z}, 1H), 6.21 (d, *J =* 7.6H_{Z}, 1H), 3.93 (s, 4H), 3.82 (s, 2H), 3.76 (d, *J =* 6.4Hz, 2H), 3.60 (d, *J =* 12.4Hz, 2H), 3.55 (s, 3H), 2.89-2.83 (m, 2H), 2.75 (s, 6H), 1.95 (m, 1H), 1.82 (d, *J =* 13.2Hz, 2H), 1.33-1.23 (m, 2H).

### Example 23. Synthesis of 1-methyl-3-((1-(oxetan-3-ylsulfonyl)piperidine-4-yl)methoxy)-6-((5-(trifluoromethyl)isoindolin-2-yl)methyl)pyridin-2(1H)-one (compound 23):

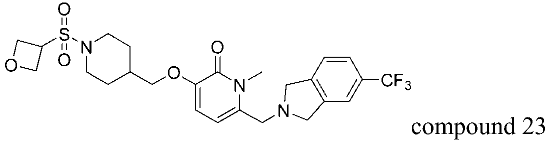

Similarly to the synthesis route of Example **1,** the above compound could be obtained by replacing the o-dichlorobenzyl in step 4 with 4-trifluoromethyl-1,2-dichlorobenzyl (intermediate 2) and replacing the methylsulfonyl chloride in step 6 with oxetane-3-sulfonyl chloride (6-4).

LC-MS [M+1]⁺ = 542.1
¹HNMR (400MHz, DMSO-d6) δ7.58 (s, 1H), 7.53 (d, *J =* 8.8Hz, 1H), 7.42 (d, *J =* 8.0 Hz, 1H), 6.75 (d, *J* = 7.6H_{Z}, 1H), 6.18 (d, *J =* 7.6H_{Z}, 1H), 4.82-4.66 (m, 5H), 3.89 (s, 4H), 3.78 (s, 2H), 3.72 (d, *J* = 6.4Hz, 2H), 3.59 (d, *J* = 12.0 Hz, 2H), 3.51 (s, 3H), 2.80-2.73 (m, 2H), 1.87-1.85 (m, 1H), 1.77 (d, *J=* 15.6Hz, 2H), 1.25-1.16 (m, 2H).

### Example 24. Synthesis of 6-((5-fluoroisoindolin-2-yl)methyl)-1-(methyl-d3)-3-((1-(methylsulfonyl)piperidine-4-yl)methoxy)pyridin-2(1H)-one (compound 24):

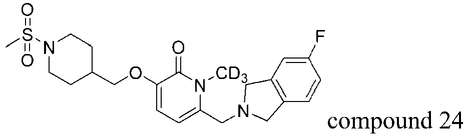

Similarly to the synthesis route of Example 1, the above compound could be obtained by replacing the iodomethane in step 1 with deuterated iodomethane and replacing the o-dichlorobenzyl in step 4 with 4-fluoro-1,2-dichlorobenzyl (intermediate 1).

LC-MS [M+1]⁺ = 453.1
¹HNMR (400MHz, DMSO-d6) δ7.26-7.22 (m, 1H), 7.10-7.07 (m, 1H), 7.02-6.98 (m, 1H), 6.79 (d, *J=* 8.0H_{Z}, 1H), 6.20 (d, *J=* 7.6H_{Z}, 1H), 3.84 (d, *J=* 11.6Hz, 4H), 3.79-3.77 (m, 4H), 3.59 (d, *J =* 11.6Hz, 2H), 2.86 (s, 3H), 2.77-2.70 (m, 2H), 1.86 (d, *J =* 13.2Hz, 3H), 1.37-1.27 (m, 2H).

### Example 25. Synthesis of 1-ethyl-6-(isoindolin-2-ylmethyl)-3-((1-(methylsulfonyl)piperidine-4-yl)methoxy)pyridin-2(1H)-one (compound 25):

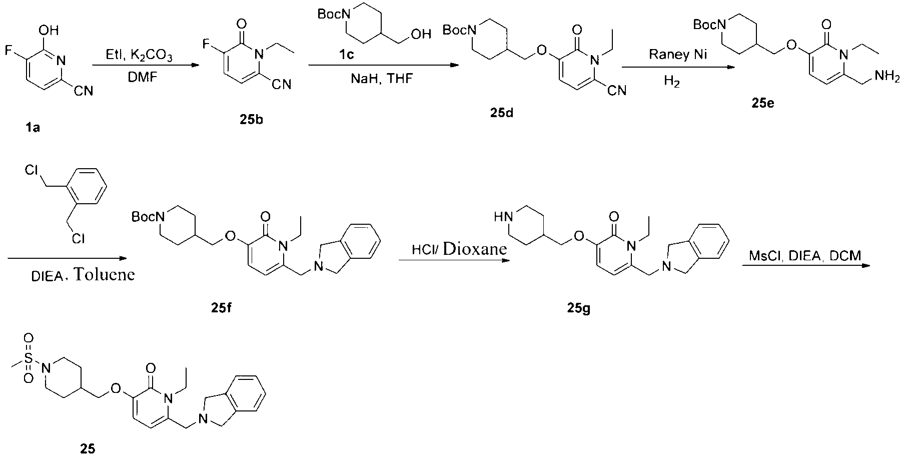

### step 1: Synthesis of 5-fluoro-1-ethyl-6-oxo-1,6-dihydropyridine-2-carbonitrile (25b):

The compound 5-fluoro-6-hydroxycyanopyridine **1a** (150 mg, 1.09 mmol) and K₂CO₃ (299.78 mg, 2.17 mmol) were dissolved in DMF (3 mL), and after iodoethane (203.29 mg, 1.30 mmol) was added, the mixture was stirred for 18 hours at 25°C. After saturated NaCl (10 mL) was added to the reaction mixture, the mixture was extracted with ethyl acetate (10 mL × 3). The organic phases were combined, dried, spin-dried, and purified by silica gel column chromatography (PE : THF = 3 : 1) to obtain compound **25b** (50.00 mg, 27.71% yield) as a white solid.

¹H-NMR (400MHz, DMSO) δ7.08 (t, *J =* 8.0 Hz, 1H), 6.74 (dd, *J* = 7.6,4.4Hz, 1H), 4.33-4.27 (m, 2H), 1.43 (s, 3H).

### step 2: Synthesis of tert-butyl 4-(((6-cyano-1-ethyl-2-oxo-1,2-dihydropyridin-3-yl)oxy)methyl)piperidine-1-carbonate (25d):

At 0°C, the compound **VN2202-022-3** (194.36 mg, 902.79 µmol) was dissolved in anhydrous THF (3 mL), and after NaH (60.19 mg, 1.50 mmol, 60% purity) was added, the mixture was stirred for 30 min at 0°C. After a solution of the compound **VN2202-022-2** (50 mg, 300.93 µmol) in anhydrous THF (1 mL) was added at 0°C, the mixture was heated to 20°C and stirred for 1 h. After quenching by adding saturated NH₄Cl (30 mL), the system was extracted three times with EA (30 mL), and the organic phases were then combined, dried, concentrated, and then purified by SGC (PE : THF = 75 : 25) to obtain the compound **VN2202-022-4** (100.00 mg, 82.75% yield) as a colorless oil.

LC-MS [M-99]⁺ = 262.20
At 0°C, the compound tert-butyl-4-(hydroxymethyl)piperidine-1-carbonate **1c** (194.36 mg, 902.79 µmol) was dissolved in THF (3 mL), and NaH (60.19 mg, 1.50 mmol, 60% purity) was slowly added and stirred for 30 minutes under nitrogen protection. At 0°C, a solution of compound **25b** (50 mg, 300.93 µmol) in anhydrous tetrahydrofuran (1 mL) was added to the reaction system, which was heated to 25°C and stirred for 1 hour under nitrogen protection. After quenching by adding saturated NH₄Cl (30 mL), the system was extracted three times with EA (30 mL), and the organic phases were combined, dried, concentrated, and then purified by silica gel column chromatography [PE : THF = 75 : 25] to obtain compound **25d** (100.00 mg, 82.75% yield) as a colorless oil.

LC-MS [M-99]⁺ = 262.20

### step 3: Synthesis of tert-butyl 4-(((6-(aminomethyl)-1-ethyl-2-oxo-1,2-dihydropyridin-3-yl)oxy)methyl)piperidine-1-carbonate (25e):

Compound **25d** (100 mg, 276.68 µmol) was dissolved in methanol (5 mL) and aqueous ammonia (1 mL), and Raney Ni (80 mg, 276.68 µmol) was added and stirred for 2 hours at 20°C. After filtration, the filtrate was concentrated to obtain compound **25e** (113.00 mg, crude) as a colorless oil.
LC-MS [M-99]⁺ = 366.3

### step 4: Synthesis of tert-butyl 4-(((1-ethyl-6-(isoindolin-2-ylmethyl)-2-oxo-1,2-dihydropyridin-3-yl)oxy)methyl)piperidine-1-carbonate (25f):

Compound **1e** (113 mg, 309.19 µmol) was dissolved in toluene (3 mL), and the compound o-dichlorobenzyl (70.36 mg, 401.95 µmol) and DIEA (153 µL, 927 µmol) were then added and stirred for 2 hours at 100°C. The reaction mixture was concentrated and extracted with ethyl acetate (30 mL × 3) and H₂O (10 mL × 3), and the organic phases were combined, dried, and concentrated. After purification by silica gel column chromatography (PE : THF = 0 : 1), compound **25f** (90.00 mg, 56.02% yield) was obtained as a white solid.
LC-MS [M+1]⁺ = 468.3

### step 5: Synthesis of 1-ethyl-6-(isoindolin-2-ylmethyl)-3-(piperidine-4-ylmethoxy)pyridin-2(1H)-one (25g):

Compound 25e (90 mg, 192.47 µmol) was dissolved in hydrochloric acid/dioxane (4M, 3 mL), and after stirring for 2 hours at 20°C, the system was concentrated to obtain compound **25g** (130.00 mg, crude) as a black solid.

### step 6: Synthesis of 1-ethyl-6-(isoindolin-2-ylmethyl)-3-((1-(methylsulfonyl)piperidine-4-yl)methoxy)pyridin-2(1H)-one (compound 25):

Compound **25g** (20.62 mg, crude) and diisopropylethylamine (21.75 mg, 168.31 µmol) were dissolved in dichloromethane (1 mL), and under ice bath condition, methylsulfonyl chloride (6.43 mg, 56.10 µmol) was dropwise added to the system, which was stirred for 30 minutes, then concentrated, and purified by semi-preparative HPLC [acetonitrile: water (0.1% ammonium bicarbonate)] to obtain compound **25** (2.64 mg, 9.83% yield) as a light brown solid.

LC-MS [M+1]⁺ = 446.1
¹HNMR (400MHz, DMSO-*d6*) δ7.20-7.14 (m, 4H), 6.74 (d, *J* = 7.6H_{Z}, 1H), 6.18 (d, *J* = 7.6H_{Z}, 1H), 4.11 (q, *J =* 6.8Hz, 2H), 3.82 (s, 4H), 3.75-3.72 (m, 4H), 3.56 (d, *J =* 11.6Hz, 2H), 2.82 (s, 3H), 2.73-2.67 (m, 2H), 1.84 (d, *J =* 11.2Hz, 3H), 1.34-1.22 (m, 2H), 1.15 (t, *J =* 6.8Hz, 3H).

### Example 26. Synthesis of 3-((1-(cyclopropylsulfonyl)piperidine-4-yl)methoxy)-1-ethyl-6-(isoindolin-2-ylmethyl)pyridin-2(1H)-one (compound 26):

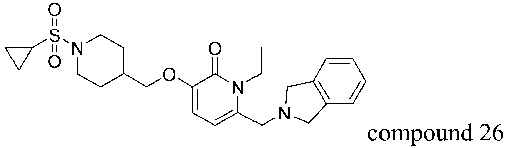

Similarly to the synthesis route of Example **25,** the above compound could be obtained by replacing the methylsulfonyl chloride in step 6 with cyclopropylsulfonyl chloride.

LC-MS [M+1]⁺ = 472.2
¹HNMR (400MHz, DMSO-d6) δ7.24-7.17 (m, 4H), 6.77 (d, *J* = 7.6H_{Z}, 1H), 6.21 (d, *J* = 7.6H_{Z}, 1H), 4.17 (q, *J =* 6.8Hz, 2H), 3.86 (s, 4H), 3.79-3.75 (m, 4H), 3.64 (d, *J =* 12.0 Hz, 2H), 2.89-2.83 (m, 2H), 2.60-2.55 (m, 1H), 1.86 (d, *J =* 12.8Hz, 3H), 1.37-1.27 (m, 2H), 1.18 (t, *J* = 6.8Hz, 3H), 1.01 (m, 2H), 0.90 (m, 2H).

### Example 27. Synthesis of 1-ethyl-6-(isoindolin-2-ylmethyl)-3-((1-((trifluoromethyl)sulfonyl)piperidine-4-yl)methoxy)pyridin-2(1H)-one (compound 27):

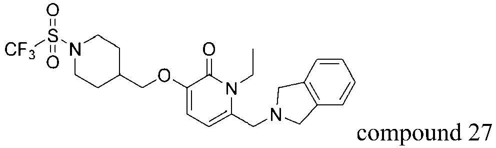

Similarly to the synthesis route of Example **25,** the above compound 27 could be obtained by replacing the methylsulfonyl chloride in step 6 with trifluoromethanesulfonic anhydride.

LC-MS [M+H]⁺ = 500.1
¹HNMR (400MHz, DMSO-d6) δ7.21-7.19 (m, 4H), 6.77 (d, *J* = 7.6H_{Z}, 1H), 6.21 (d, *J* = 7.6H_{Z}, 1H), 4.14 (q, *J =* 6.8Hz, 2H), 3.85 (s, 6H), 3.77 (m, 4H), 3.26-3.20 (m, 2H), 2.07-2.05 (m, 1H), 1.93-1.89 (m, 2H), 1.37-1.27 (m, 2H), 1.18 (t, *J* = 6.8Hz, 3H).

### Example 28. Synthesis of 4-(((1-ethyl-6-(isoindolin-2-ylmethyl)-2-oxo-1,2-dihydropyridin-3-yl)oxy)methyl)piperidine-1-sulfonamide (compound 28) and 4-(((1-ethyl-6-(isoindolin-2-ylmethyl)-2-oxo-1,2-dihydropyridin-3-yl)oxy)methyl)piperidine-1-sulfonic acid (compound 31):

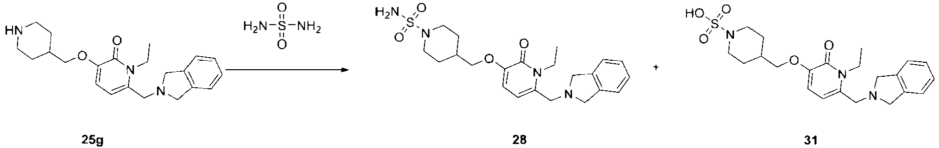

Compound **25g** (3.8 g, 4.31 mmol), the compound sulfamide (1.66 g, 17.26 mmol), and DIEA (3.35 g, 25.89 mmol) were successively added to a mixed solution of dioxane (70 mL) and acetonitrile (30 mL). The mixture was heated to 100°C and stirred for 16 h. After cooling, the mixture was concentrated to remove the solvent and purified by SGC (DCM : MeOH = 97 : 3-85 : 15) to obtain crude **28** (800 mg) and crude **31** (200 mg).

28 (800 mg, crude) was added to a 3M hydrochloric acid aqueous solution (80 mL), and dichloromethane (80 mL) was slowly added under stirring, during which a solid precipitated. After filtration, the filter cake was dried to obtain **28** hydrochloride (210.00 mg, 99.37% purity) as a light green solid. After the mother liquor was layered, the aqueous phase was adjusted to pH-8 with sodium bicarbonate and extracted twice with dichloromethane (80 mL). The organic phases were combined, dried, and concentrated to obtain a solid, which was then purified by Prep. HPLC to obtain **28** free base (270.00 mg, 13.81% yield, 98.55% purity) as a white solid.

Crude 31 (200 mg) was purified by Prep. HPLC to obtain **31** (30.00 mg, 99.23% purity) as a white solid (trifluoroacetate).

The data of compound 28 free base were as follow:
LC-MS [M+H]⁺ = 447.1
¹HNMR (400MHz, DMSO-d6) δ7.24-7.19 (m, 4H), 6.77 (d, *J* = 7.6H_{Z}, 1H), 6.73 (s, 2H), 6.21 (d, *J =* 7.6H_{Z}, 1H), 4.14 (q, *J =* 6.8Hz, 2H), 3.85 (s, 4H), 3.79 (s, 2H), 3.74 (d, *J =* 6.0 Hz, 2H), 3.52-3.49 (m, 2H), 2.56-2.50 (m, 2H), 1.87-1.78 (m, 3H), 1.37-1.30 (m, 2H), 1.18 (t, *J =* 6.8Hz, 3H).

The data of compound 28 hydrochloride were as follow:
1H-NMR (400MHz, DMSO-d6) δ7.37-7.36 (m, 4H), 6.84 (d, *J =* 7.6Hz, 1H), 6.54 (d, *J* = 7.6Hz, 1H), 4.62-4.58 (m, 6H), 4.07-4.05 (m, 2H), 3.82-3.76 (m, 2H), 3.46 (d, *J =* 10.8Hz, 2H), 2.54-2.51 (m, 2H), 1.85-1.82 (m, 3H), 1.36-1.26 (m, 2H), 1.12 (t, *J* = 6.8Hz, 3H).

The data of compound 31 trifluoroacetate were as follow:
LC-MS [M+H]⁺ = 448.2

¹H-NMR (400MHz, DMSO-d6) δ7.23-7.19 (m, 4H), 6.78 (d, *J* = 7.6Hz, 1H), 6.26 (s, 1H), 4.13-3.56 (m, 12H), 3.50-3.35 (m, 2H), 2.82 (s, 1H), 1.96-1.90 (m, 3H), 1.43-1.33 (m, 2H), 1.15 (t, *J* = 7.2Hz, 3H).

### Example 29. Synthesis of 4-(((1-ethyl-6-(isoindolin-2-ylmethyl)-2-oxo-1,2-dihydropyridin-3-yl)oxy)methyl)-N-methylpiperidine-1-sulfonamide (compound 29):

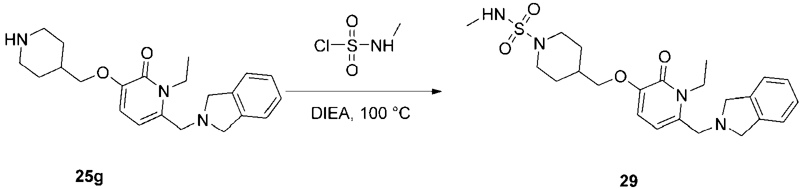

Under ice bath condition, **25g** (50 mg, 113.53 µmol, crude), methylsulfamoyl chloride (16.18 mg, 124.88 µmol), and diisopropylethylamine (44.02 mg, 340.60 µmol) were added to dichloromethane (2 mL). After the addition was complete, the mixture was stirred for 30 min. The reaction mixture was directly concentrated and subjected to preparation by Prep. HPLC to obtain the desired product **29** (2.18 mg, 4.39 µmol, 3.87% yield) as a white solid.

LC-MS [M+H]⁺ = 461.3
¹HNMR (400MHz, DMSO-d6) δ7.24-7.17 (m, 4H), 7.04 (q, *J* = 4.2Hz, 1H), 6.77 (d, *J* = 7.6H_{Z}, 1H), 6.20 (d, *J* = 7.6H_{Z}, 1H), 4.14 (q, *J* = 6.8Hz, 2H), 3.86 (s, 4H), 3.79 (s, 2H), 3.74 (d, *J* = 6.0 Hz, 2H), 3.54 (d, *J =* 11.6Hz, 2H), 2.73-2.67 (m, 2H), 2.52-2.50 (m, 3H), 1.90-1.82 (m, 3H), 1.33-1.24 (m, 2H), 1.17 (t, *J* = 6.8Hz, 3H).

### Example 30. Synthesis of 4-(((1-ethyl-6-(isoindolin-2-ylmethyl)-2-oxo-1,2-dihydropyridin-3-yl)oxy)methyl)-N-sulfamoylpiperidine-1-sulfonamide (compound 30):

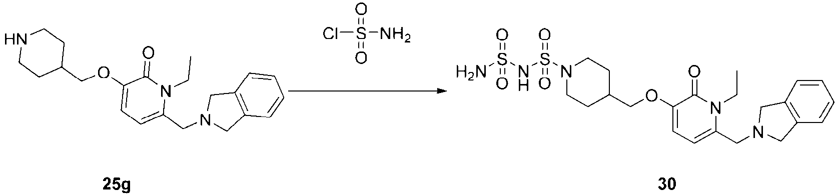

Under ice bath, compound **25g** (800 mg, 2.18 mmol) and DIEA (844.07 mg, 6.53 mmol) were successively dissolved in DCM (30 mL), and after the compound aminosulfonyl chloride (301.83 mg, 2.61 mmol) was added to the system, the system was stirred for 2 h. 1M hydrochloric acid aqueous solution (30 mL) was added, and the system was extracted three times with dichloromethane (50 mL). The organic phases were combined, dried, concentrated, and purified by SGC (DCM : MeOH = 5 : 1) and then by Prep. HPLC to obtain **30** (30.00 mg, 2.58% yield) as a white solid.

LC-MS [M+1]⁺ = 526.2
¹H-NMR (400MHz, DMSO-d6) δ7.26-7.24 (m, 4H), 6.78 (d, *J =* 7.6Hz, 1H), 6.29 (s, 1H), 4.71-3.90 (m, 8H), 3.73 (d, *J =* 6.0 Hz, 2H), 3.52 (d, *J =* 12.0 Hz, 2H), 2.67 (d, *J =* 11.6Hz, 2H), 1.80-1.78 (m, 3H), 1.32-1.23 (m, 2H), 1.15 (t, *J =* 7.2Hz, 3H).

### Test Example 1

Conversion of cholesterol into pregnenolone is a first rate-limiting step in the synthesis of all steroid hormones in vertebrates. This conversion reaction involves a two-step continuous monooxygenation reaction catalyzed by the unique cytochrome P450scc (CYP11A) to produce C₂₂-hydroxycholesterol and C₂₀,C₂₂-dihydroxycholesterol in order, followed by CYP11A1-catalyzed breakage of a C₂₀-C₂₂ carbon-carbon single bond in C₂₀,C₂₂-dihydroxycholesterol to produce one molecule of pregnenolone and one molecule of 4-methylvaleraldehyde. Therefore, the inhibitory effect of a compound on CYP11A1 was identified by detecting the ability of the compound to inhibit pregnenolone synthesis.

The ability of different compounds to inhibit pregnenolone biosynthesis was determined by detecting the concentration of pregnenolone (Abnova pregnenolone ELISA kit, KA1912) using enzyme-linked immunosorbent assay (ELISA), so as to detect the ability of the compounds to inhibit CYP11A1. The human adrenocortical cancer cell line NCI-H295R (Procell, CL-0399) has been demonstrated to express all key steroid synthetases and was thus used as an enzyme source. In order to determine the half-maximal inhibitory concentrations (IC50) of CYP11A1 inhibition by different compounds, the compounds were added during NCI-H295R cell culture, and after incubation, the concentration of pregnenolone in the culture supernatant was determined.

95 µl of NCI-H295R cells were taken and cultured overnight with a specialized cell culture medium (Procell, CM-0399) in a 96-well culture plate in a cell incubator at 37°C and 5% CO₂. 5 µl/well of a DMSO (Sigma, D8418) solution of the compounds to be tested at different concentrations was then added, followed by continued incubation for 24 hours. The final concentrations of the compounds to be tested were 1000 nM, 333.33 nM, 111.11 nM, 37.04 nM, 12.35 nM, 4.12 nM, 1.37 nM, 0.46 nM, and 0 nM. After the incubation was complete, the cells were centrifuged, 80 µl/well of the cell culture supernatant was obtained and 1 : 8 diluted with the cell culture medium, and the concentration of pregnenolone therein was then determined by ELISA method. During ELISA detection, a pregnenolone standard was used to generate a standard curve. The experiment was carried out with replicate wells. The ELISA well plate had been coated with an anti-pregnenolone rabbit polyclonal antibody. In ELISA plate strips, 50 µl/well of the diluted culture supernatant or standard solution or reference substance was added, followed by 100 µl of a pregnenolone-HRP complex solution, and the plate was incubated for 1 hour at room temperature on a plate shaker (QILINBEIER, QB-9002) at 200 rpm. Then, the plate was washed three times with a cleaning solution, with 300 µl each time, and patted dry on absorbent paper. 150 µl of a TMB substrate was added. The plate was incubated on the plate shaker for 10-15 minutes at room temperature, and 50 µl of a stop solution was then added. The absorbance was measured at 450 nm by a microplate reader (PerkinElmer, 2105). The IC₅₀ value was calculated by using GraphPad Prism software. Inhibition rate (%) = 100% - (Readoutcompound - Average readoutpositive control) / (Average readoutblank control - Average readoutpositive control) x 100%.

| Compound | Pregnenolone synthesis inhibition IC50 (nM) | Compound | Pregnenolone synthesis inhibition IC50 (nM) |
|---|---|---|---|
| 1 | 4.15 | 16 | 1.030 |
| 2 | 221.8 | 17 | 3.756 |
| 3 | 2.255 | 19 | 7.796 |
| 4 | 1.738 | 20 | 1.942 |
| 5 | 8.49 | 21 | 2.206 |
| 6 | 1.938 | 22 | 8.613 |
| 7 | 9.343 | 23 | 3.573 |
| 8 | 4.524 | 24 | 4.860 |
| 11 | 1.977 | 25 | 1.561 |
| 12 | 3.573 | 26 | 0.700 |
| 13 | 11.96 | 27 | 0.136 |
| 14 | 4.753 | 28 | 1.181 |
| 15 | 3.988 | 29 | 14.79 |
| | | 30 | 0.648 |

### Test Example 2

The ability of different compounds to inhibit testosterone biosynthesis was determined by detecting the concentration of testosterone (Abnova testosterone ELISA kit, KA6502) using enzyme-linked immunosorbent assay (ELISA), so as to detect the ability of the compounds to inhibit CYP11A1. In the experiment, similarly, the human adrenocortical cancer cell line NCI-H295R (Procell, CL-0399) was also used as an enzyme source. The compounds were added during NCI-H295R cell culture. After incubation, the concentration of testosterone in the culture supernatant was determined.

95 µl of NCI-H295R cells were taken and cultured overnight with a specialized cell culture medium (Procell, CM-0399) in a 96-well culture plate in a cell incubator at 37°C and 5% CO₂. 5 µl/well of a DMSO (Sigma, D8418) solution of the compounds to be tested at different concentrations was then added, followed by continued incubation for 24 hours. The final concentrations of the compounds to be tested were 1000 nM, 333.33 nM, 111.11 nM, 37.04 nM, 12.35 nM, 4.12 nM, 1.37 nM, 0.46 nM, and 0 nM. After the incubation was complete, the cells were centrifuged, 80 µl/well of the cell culture supernatant was obtained, and the concentration of testosterone therein was determined by ELISA method. During ELISA detection, a testosterone standard was used to generate a standard curve. The experiment was carried out with replicate wells. The ELISA well plate had been coated with an anti-testosterone monoclonal antibody. In ELISA plate strips, 25 µl/well of the culture supernatant or standard solution or reference substance was added, followed by 200 µl of a testosterone-HRP complex solution, and the plate was incubated for 1 hour at room temperature on a plate shaker (QILINBEIER, QB-9002) at 200 rpm. Then, the plate was washed three times with a cleaning solution, with 300 µl each time, and patted dry on absorbent paper. 200 µl of a TMB substrate was added. The plate was incubated on the plate shaker for 15 minutes at room temperature, and 100 µl of a stop solution was then added. The absorbance was measured at 450 nm by a microplate reader (PerkinElmer, 2105). The IC₅₀ value was calculated by using GraphPad Prism software. Inhibition rate (%) = 100% - (Readoutcompound - Average readoutpositive control) / (Average readoutblank control - Average readoutpositive control) x 100%.

| Compound | Testosterone synthesis inhibition IC50 (nM) |
|---|---|
| 1 | 17.7 |
| 16 | 40.23 |
| 25 | 3.778 |
| 26 | 2.454 |
| 28 | 7.023 |
| 30 | 4.044 |
| 31 | > 1000 |

### Test Example 3. Pharmacokinetic evaluation

Comparative Example 1, Example 185 was synthesized by referencing patent WO 2018115591 A1, and the structural formula was as follows:

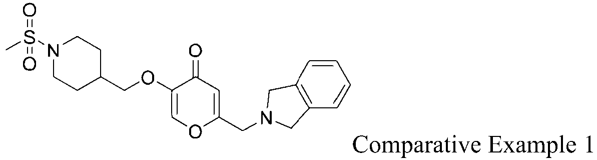

### ICR mouse experiment

### 1. Summary

Taking ICR mice as test animals, the drug concentration in plasma of ICR mice was determined by LC/MS/MS method at different times after intragastric administration (i.g.) with the compound of the present disclosure. The pharmacokinetic behavior of the compound of the present disclosure in ICR mice was studied and the pharmacokinetic characteristics thereof were evaluated.

### 2. Experimental protocol

### 2.1 Experimental drug

### Compound of Example 1

### Compound of Example 28

### Compound of Comparative Example 1

### 2.2 Experimental animals

27 male ICR mice were taken and randomly divided into three groups. The mice were provided by JOINN Laboratories (Suzhou) Co., Ltd. Before administration, the mice were fasted overnight for at least 12 hours. During the fasting and experiment, the drinking water was normal saline.

### 2.3 Drug formulation

A certain amount of the compound to be tested was taken, and the test sample prepared with a final concentration of 2 mg/mL was taken for oral administration. The preparation solvent was 0.5% Tween 80 in a 0.5% methylcellulose aqueous solution. After the preparation was complete, a 1N hydrochloric acid aqueous solution was added for adjustment to a clear solution (pH 4-5).

### 2.4 Administration

The dosage was 20.0 mg/kg and the dosage volume was 10.0 ml/kg.

### 3. Procedure

At 15 min, 30 min, 1 h, 2 h, 4 h, 6 h, 8 h, 10 h, and 24 h after administration, at least 0.2 mL was taken from the vein (3 animals at each time point) and placed in an EDTA-K₂ anticoagulation test tube. The blood sample was collected and then placed in a labeled ice-water bath centrifuge tube, and the plasma was quickly separated by centrifugation. The centrifugation conditions were as follows: 4000 revolutions/minute, 10 minutes, and 4°C. The plasma was stored at -40°C or lower for later testing. Feed supply was resumed 4 hours after administration.

Determination of the content of the compound to be detected in the plasma of ICR mice: After the plasma sample was thawed at room temperature, 50 µL was taken and added to 300 µL of (200 ng/mL, acetonitrile, terfenadine) internal standard, the mixture was vortexed and uniformly mixed for 1 min and then centrifuged at 4°C at 15400 g for 10 min. The supernatant was taken and injected for LC/MS/MS analysis.

### 4. Data collection and statistical analysis

By Analyst 1.6.3 software, data such as original chromatogram, concentration, and accuracy were output.

By Microsoft Excel 2007 software, the average value, standard deviation, coefficient of variation, etc., were calculated.

The main pharmacokinetic parameters, such as AUC, Cₘₐₓ and t_{1/2}, were calculated by non-compartmental analysis (NCA) method using WinNonlin software.

### 5. Results of pharmacokinetic parameters

| Compound No. | Mode of administration /Dosage (mg/kg) | Plasma concentration Cmax (ng/mL) | Area under the curve AUC₀₋ₜ (h*ng/mL) | Half-life T_{1/2} (h) |
|---|---|---|---|---|
| Comparative Example 1 | i.g./20 | 7947 | 4331 | 1.87 |
| Example 1 | i.g./20 | 9723 | 9951 | 0.708 |
| Example 28 | i.g./20 | 5443 | 13643 | 1.22 |

The above description is only for specific embodiments of the present disclosure and the scope of protection of the present disclosure is not limited thereto. Variations or substitutions readily conceivable to any of those who are familiar with the technical field within the technical scope disclosed by the present disclosure should all be covered by the scope of protection of the present disclosure. Therefore, the scope of protection of the present disclosure should be based on the scope of protection of the claims.

## Claims

1. A compound represented by general formula (I), or a stereoisomer, tautomer, deuterated derivative or pharmaceutically acceptable salt thereof: 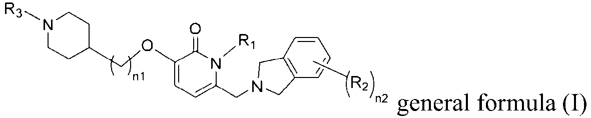
R₁ is selected from a hydrogen atom, C₁₋₆ alkyl, C₃₋₆ cycloalkyl, C₆₋₁₄ aryl, 4- to 8-membered heterocyclyl containing 1 to 3 heteroatoms selected from N, O, and S, 5- to 8-membered heteroaryl containing 1 to 3 heteroatoms selected from N, O, and S, wherein the C₁₋₆ alkyl, C₃₋₆ cycloalkyl, C₆₋₁₄ aryl, 4- to 8-membered heterocyclyl, and 5- to 8-membered heteroaryl are optionally substituted with one or more Rₐ substituents,
each Rₐ is the same as or different from each other and is independently selected from hydrogen, deuterium, tritium, halogen, amino, hydroxyl, cyano, C₁₋₆ alkoxy, C₁₋₆ alkyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy;
R₂ is selected from a hydrogen atom, a halogen atom, C₁₋₆ alkyl, C₂₋₆ alkenyl, C₂₋₆ alkynyl, C₁₋₆ alkoxy, C₃₋₆ cycloalkyl, C₆₋₁₄ aryl, 4- to 8-membered heterocyclyl containing 1 to 3 heteroatoms selected from N, O, and S, 5- to 8-membered heteroaryl containing 1 to 3 heteroatoms selected from N, O, and S, wherein the C₁₋₆ alkyl, C₂₋₆ alkenyl, C₂₋₆ alkynyl, C₁₋₆ alkoxy, C₃₋₆ cycloalkyl, C₆₋₁₄ aryl, 4- to 8-membered heterocyclyl, and 5- to 8-membered heteroaryl are optionally substituted with one or more R_{b} substituents,
each R_{b} is the same as or different from each other and is independently selected from hydrogen, deuterium, tritium, halogen, amino, hydroxyl, cyano, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy;
R₃ is selected from C₁₋₇ alkylcarbonyl, C₂₋₇ alkenylcarbonyl, C₂₋₇ alkynylcarbonyl, C₁₋₇ alkoxycarbonyl, C₃₋₇ cycloalkylcarbonyl, 3- to 8-membered heterocycloalkylcarbonyl containing 1 to 3 heteroatoms selected from N, O, and S, NR_{c}R_{d} carbonyl, sulfonic acid group, aminosulfonyl C₁₋₇ alkyl S(O)₂-, C₂₋₇ alkenyl S(O)₂-, C₂₋₇ alkynyl S(O)₂-, C₁₋₇ alkoxy S(O)₂-, C₃₋₇ cycloalkyl S(O)₂-, 3- to 8-membered heterocycloalkyl S(O)₂- containing 1 to 3 heteroatoms selected from N, O, and S, NR_{c}R_{d}S(O)₂-, wherein the C₁₋₇ alkylcarbonyl, C₂₋₇ alkenylcarbonyl, C₂₋₇ alkynylcarbonyl, C₁₋₇ alkoxycarbonyl, C₃₋₇ cycloalkylcarbonyl, 3- to 8-membered heterocycloalkylcarbonyl, C₁₋₇ alkyl S(O)₂-, C₂₋₇ alkenyl S(O)₂-, C₂₋₇ alkynyl S(O)₂-, C₁₋₇ alkoxy S(O)₂-, C₃₋₇ cycloalkyl S(O)₂-, 3- to 8-membered heterocycloalkyl S(O)₂- are optionally substituted with one or more Rₑ substituents;
R_{c} and R_{d} are each independently selected from hydrogen, C₁₋₆ alkoxy, C₁₋₆ alkyl, C₂₋₆ alkenyl, C₂₋₆ alkynyl, C₃₋₆ cycloalkyl, and aminosulfonyl, or R_{c} and R_{d}, together with the connected N atom, form a 3- to 6-membered heterocyclic ring;
each Rₑ is the same as or different from each other and is independently selected from hydrogen, deuterium, tritium, halogen, amino, hydroxyl, cyano, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl containing 1 to 3 heteroatoms selected from N, O, and S, wherein the amino, C₁₋₆ alkoxy, C₁₋₆ alkyl, C₂₋₆ alkenyl, C₂₋₆ alkynyl, C₃₋₆ cycloalkyl, 4- to 8-membered heterocycloalkyl are optionally substituted with 1 to 3 substituents selected from C₁₋₆ alkyl, halo C₁₋₆ alkyl, halogen, amino, hydroxyl, cyano, or C₁₋₆ alkoxy;
n1 is an integer of 0, 1, 2, 3, or 4;
n2 is an integer of 1, 2, 3, or 4.

2. The compound represented by general formula (I), or the stereoisomer, tautomer, deuterated derivative or pharmaceutically acceptable salt thereof according to claim 1, which **characterized in that**, R₁ is selected from a hydrogen atom, C₁₋₄ alkyl, C₃₋₆ cycloalkyl, C₆₋₁₀ aryl, 4- to 6-membered heterocyclyl containing 1 to 3 heteroatoms selected from N, O, and S, 5- to 6-membered heteroaryl containing 1 to 3 heteroatoms selected from N, O, and S, wherein the C₁₋₄ alkyl, C₃₋₆ cycloalkyl, C₆₋₁₀ aryl, 4- to 6-membered heterocyclyl and 5- to 6-membered heteroaryl are optionally substituted with 1 to 3 Rₐ substituents,
each Rₐ is the same as or different from each other and is independently selected from hydrogen, deuterium, tritium, halogen, amino, hydroxyl, cyano, C₁₋₄ alkoxy, C₁₋₄ alkyl, C₃₋₆ cycloalkyl, 4- to 6-membered heterocycloalkyl containing 1 to 3 heteroatoms selected from N, O, and S;
preferably, R₂ is selected from a hydrogen atom, a halogen atom, C₁₋₄ alkyl, C₂₋₄ alkenyl, C₂₋₄ alkynyl, C₁₋₄ alkoxy, C₃₋₆ cycloalkyl, C₆₋₁₀ aryl, 4- to 6-membered heterocyclyl containing 1 to 3 heteroatoms selected from N, O, S, 5- to 6-membered heteroaryl containing 1 to 3 heteroatoms selected from N, O, and S, wherein the C₁₋₄ alkyl, C₂₋₄ alkenyl, C₂₋₄ alkynyl, C₁₋₄ alkoxy, C₃₋₆ cycloalkyl, C₆₋₁₀ aryl, 4- to 6-membered heterocyclyl and 5- to 6-membered heteroaryl are optionally substituted with one or more R_{b} substituents,
each R_{b} is the same as or different from each other and is independently selected from hydrogen, deuterium, tritium, halogen, amino, hydroxyl, cyano, C₁₋₄ alkoxy, C₁₋₄ alkyl, C₂₋₄ alkenyl, C₂₋₄ alkynyl, C₃₋₆ cycloalkyl, 4- to 6-membered heterocycloalkyl containing 1 to 3 heteroatoms selected from N, O, and S;
preferably, R₃ is selected from C₁₋₄ alkylcarbonyl, C₂₋₄ alkenylcarbonyl, C₂₋₄ alkynylcarbonyl, C₁₋₄ alkoxycarbonyl, C₃₋₆ cycloalkylcarbonyl, sulfonic acid group, aminosulfonyl, 3- to 6-membered heterocycloalkylcarbonyl containing 1 to 3 heteroatoms selected from N, O, and S, NR_{c}R_{d} carbonyl, C₁₋₄ alkyl S(O)₂-, C₂₋₄ alkenyl S(O)₂-, C₂₋₄ alkynyl S(O)₂-, C₁₋₄ alkoxy S(O)₂-, C₃₋₆ cycloalkyl S(O)₂-, 3- to 6-membered heterocycloalkyl S(O)₂- containing 1 to 3 heteroatoms selected from N, O, and S, NR_{c}R_{d}S(O)₂-, wherein the C₁₋₄ alkylcarbonyl, C₂₋₄ alkenylcarbonyl, C₂₋₄ alkynylcarbonyl, C₁₋₄ alkoxycarbonyl, C₃₋₆ cycloalkylcarbonyl, 3- to 6-membered heterocycloalkylcarbonyl, C₁₋₄ alkyl S(O)₂-, C₂₋₄ alkenyl S(O)₂-, C₂₋₄ alkynyl S(O)₂-, C₁₋₄ alkoxy S(O)₂-, C₃₋₆ cycloalkyl S(O)₂-, 3- to 6-membered heterocycloalkyl S(O)₂- are optionally substituted with 1 to 3 Rₑ substituents; and
R_{c} and R_{d} are each independently selected from hydrogen, C₁₋₄ alkoxy, C₁₋₄ alkyl, C₂₋₄ alkenyl, C₂₋₄ alkynyl, C₃₋₆ cycloalkyl, and aminosulfonyl, or R_{c} and R_{d}, together with the connected N atom, form a 3- to 6-membered heterocyclic ring; and
each Rₑ is the same as or different from each other and is independently selected from hydrogen, deuterium, tritium, halogen, amino, hydroxyl, cyano, C₁₋₄ alkoxy, C₁₋₄ alkyl, C₂₋₄ alkenyl, C₂₋₄ alkynyl, C₃₋₆ cycloalkyl, 4- to 6-membered heterocycloalkyl containing 1 to 3 heteroatoms selected from N, O, and S.

3. The compound represented by general formula (I), or the stereoisomer, tautomer, deuterated derivative or pharmaceutically acceptable salt thereof according to claim 1, which **characterized in that**, R₁ is selected from a hydrogen atom, C₁₋₃ alkyl, C₃₋₆ cycloalkyl, 4- to 6-membered heterocyclyl containing 1 or 2 heteroatoms selected from N, O, and S, 5- to 6-membered heteroaryl containing 1 or 2 heteroatoms selected from N, O, and S, wherein the C₁₋₃ alkyl, C₃₋₆ cycloalkyl, 4- to 6-membered heterocyclyl, and 5- to 6-membered heteroaryl are optionally substituted with 1 or 2 Rₐ substituents, and
each Rₐ is the same as or different from each other and is independently selected from hydrogen, deuterium, tritium, halogen, amino, hydroxyl, cyano, C₁₋₃ alkoxy, C₁₋₃ alkyl, C₃₋₆ cycloalkyl;
preferably, R₂ is selected from a hydrogen atom, a halogen atom, C₁₋₃ alkyl, C₂₋₃ alkenyl, C₂₋₃ alkynyl, C₁₋₃ alkoxy, C₃₋₆ cycloalkyl, C₆₋₁₀ aryl, 4- to 6-membered heterocyclyl containing 1 or 2 heteroatoms selected from N, O, and S, 5- to 6-membered heteroaryl containing 1 or 2 heteroatoms selected from N, O, and S, wherein the C₁₋₃ alkyl, C₂₋₃ alkenyl, C₂₋₃ alkynyl, C₁₋₃ alkoxy, C₃₋₆ cycloalkyl, C₆₋₁₀ aryl, 4- to 6-membered heterocyclyl and 5- to 6-membered heteroaryl are optionally substituted with one or more R_{b} substituents, and
each R_{b} is the same as or different from each other and is independently selected from hydrogen, deuterium, tritium, halogen, amino, hydroxyl, cyano, C₁₋₃ alkoxy, C₁₋₃ alkyl, C₂₋₃ alkenyl, C₂₋₃ alkynyl, C₃₋₆ cycloalkyl;
preferably, R₃ is selected from C₁₋₃ alkylcarbonyl, C₂₋₃ alkenylcarbonyl, C₂₋₃ alkynylcarbonyl, C₁₋₃ alkoxycarbonyl, C₃₋₆ cycloalkylcarbonyl, sulfonic acid group, aminosulfonyl, 3- to 6-membered heterocycloalkylcarbonyl containing 1 or 2 heteroatoms selected from N, O, and S, NR_{c}R_{d} carbonyl, C₁₋₃ alkyl S(O)₂-, C₂₋₃ alkenyl S(O)₂-, C₂₋₃ alkynyl S(O)₂-, C₁₋₃ alkoxy S(O)₂-, C₃₋₆ cycloalkyl S(O)₂-, 3- to 6-membered heterocycloalkyl S(O)₂- containing 1 or 2 heteroatoms selected from N, O, and S, NR_{c}R_{d}S(O)₂-, wherein the C₁₋₃ alkylcarbonyl, C₂₋₃ alkenylcarbonyl, C₂₋₃ alkynylcarbonyl, C₁₋₃ alkoxycarbonyl, C₃₋₆ cycloalkylcarbonyl, 3- to 6-membered heterocycloalkylcarbonyl, C₁₋₃ alkyl S(O)₂-, C₂₋₃ alkenyl S(O)₂-, C₂₋₃ alkynyl S(O)₂-, C₁₋₃ alkoxy S(O)₂-, C₃₋₆ cycloalkyl S(O)₂-, 3- to 6-membered heterocycloalkyl S(O)₂- are optionally substituted with 1 or 2 Rₑ substituents; and
R_{c} and Rₐ are each independently selected from hydrogen, C₁₋₄ alkoxy, C₁₋₄ alkyl, C₃₋₆ cycloalkyl, aminosulfonyl, or R_{c} and R_{d}, together with the connected N atom, form a 3- to 6-membered heterocyclic ring; and
each Rₑ is the same as or different from each other and is independently selected from hydrogen, deuterium, tritium, halogen, amino, hydroxyl, cyano, C₁₋₃ alkoxy, C₁₋₃ alkyl, C₃₋₆ cycloalkyl.

4. The compound represented by general formula (I), or the stereoisomer, tautomer, deuterated derivative or pharmaceutically acceptable salt thereof according to claim 1, which **characterized in that**, R₁ is selected from a hydrogen atom, methyl, ethyl, n-propyl, isopropyl, cyclopropyl, deuterated methyl, deuterated ethyl, deuterated n-propyl, deuterated isopropyl, and deuterated cyclopropyl;
preferably, R₂ is selected from a hydrogen atom, fluorine, chlorine, bromine, methyl, ethyl, n-propyl, isopropyl, monofluoromethyl, difluoromethyl, trifluoromethyl, dichloromethyl, trichloromethyl, monofluoroethyl, difluoroethyl, trifluoroethyl, tetrafluoroethyl, pentafluoroethyl, dichloroethyl, trichloroethyl, tetrachloroethyl, pentachloroethyl, difluoropropyl, trifluoropropyl, tetrafluoropropyl, pentafluoropropyl, hexafluoropropyl, perfluoropropyl, monochloropropyl, dichloropropyl, trichloropropyl, tetrachloropropyl, pentachloropropyl, hexachloropropyl, and perchloropropyl;
preferably, R₃ is selected from a hydrogen atom, methyl-S(O)₂-, ethyl-S(O)₂-, n-propylS(O)₂-, isopropyl-S(O)₂-, cyclopropyl-S(O)₂-, oxiranyl-S(O)₂-, cyclobutyl-S(O)₂-, oxetanyl-S(O)₂-, methoxy-S(O)₂-, ethoxy-S(O)₂-, n-propoxy-S(O)₂-, isopropoxy-S(O)₂-, cyclopropoxy-S(O)₂-, oxiranyloxy-S(O)₂-, cyclobutoxy-S(O)₂-, oxetanyloxy-S(O)₂-, N,N-dimethylamino-S(O)₂-, trifluoromethyl-S(O)₂-, amino-S(O)₂-, SO₃H-, pyrroline-1-S(O)₂-, piperidine-1-S(O)₂-, morpholine-1-S(O)₂-, methylcarbonyl, ethylcarbonyl, n-propylcarbonyl, isopropylcarbonyl, cyclopropylcarbonyl, oxiranylcarbonyl, cyclobutylcarbonyl, oxetanylcarbonyl, methoxycarbonyl, ethoxycarbonyl, n-propoxycarbonyl, isopropoxycarbonyl, cyclopropoxycarbonyl, oxiranyloxycarbonyl, cyclobutoxycarbonyl, oxetanyloxycarbonyl, N,N-dimethylaminocarbonyl, trifluoromethylcarbonyl, and 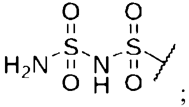
preferably, n1 is 1;
preferably, n2 is 1.

5. The compound represented by general formula (I), or the stereoisomer, tautomer, deuterated derivative or pharmaceutically acceptable salt thereof according to claim 1, which **characterized in that**, the compound represented by general formula (I), or the stereoisomer, tautomer, deuterated derivative or pharmaceutically acceptable salt thereof is one of the following compounds:

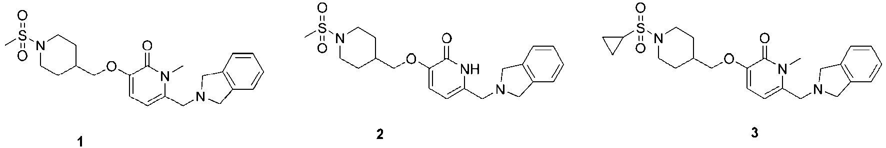

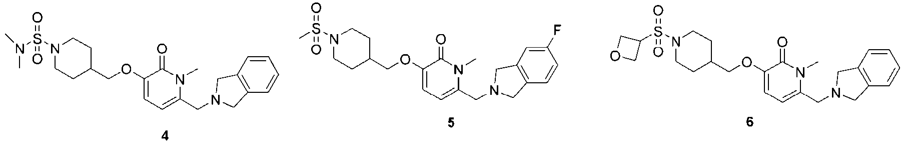

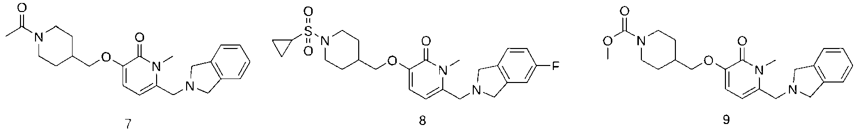

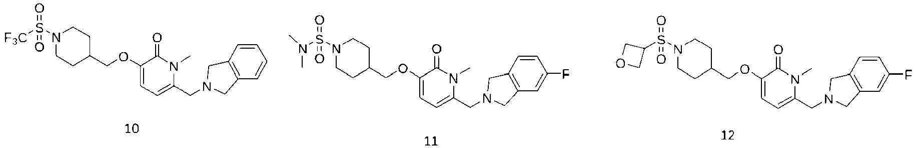

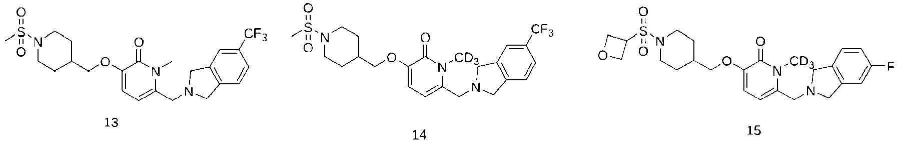

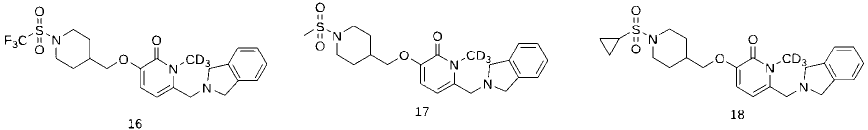

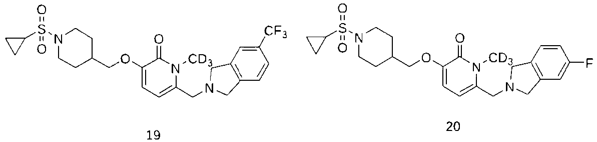

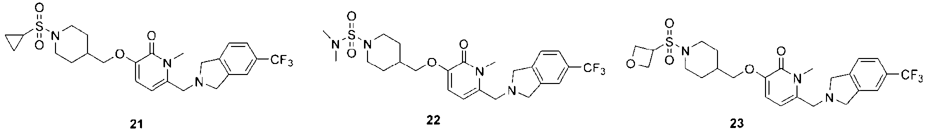

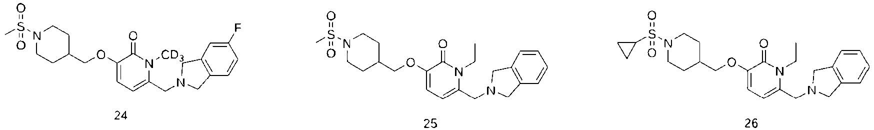

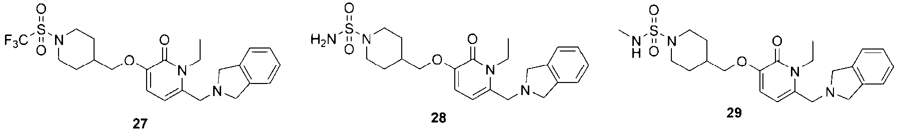

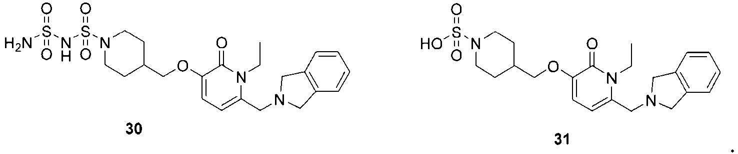

6. Use of the compound represented by general formula (I), or the stereoisomer, tautomer, deuterated derivative or pharmaceutically acceptable salt thereof according to any one of claims 1 to 5 as a CYP11A1 inhibitor.

7. Use of the compound represented by general formula (I), or the stereoisomer, tautomer, deuterated derivative or pharmaceutically acceptable salt thereof according to any one of claims 1 to 5 in the preparation of a drug for treating a steroid hormone-dependent disease.

8. Use of the compound represented by general formula (I), or the stereoisomer, tautomer, deuterated derivative or pharmaceutically acceptable salt thereof according to any one of claims 1 to 5 in the preparation of a drug for treating a steroid receptor-dependent disease such as prostate cancer or breast cancer.

9. A pharmaceutical composition, the pharmaceutical composition comprising a therapeutically effective amount of the compound represented by general formula (I), or the stereoisomer, tautomer, deuterated derivative or pharmaceutically acceptable salt thereof according to any one of claims 1 to 5 as an active ingredient, and a pharmaceutically acceptable excipient.

10. A method for treating prostate cancer, the method comprises administering a therapeutically effective amount of the compound represented by general formula (I), or the stereoisomer, tautomer, deuterated derivative or pharmaceutically acceptable salt thereof according to any one of claims 1 to 5, or the pharmaceutical composition according to claim 9 to a prostate cancer patient in need thereof.

11. A method for treating breast cancer, the method comprises administering a therapeutically effective amount of the compound represented by general formula (I), or the stereoisomer, tautomer, deuterated derivative or pharmaceutically acceptable salt thereof according to any one of claims 1 to 5, or the pharmaceutical composition according to claim 9 to a breast cancer patient in need thereof.

12. The compound represented by general formula (I), or the stereoisomer, tautomer, deuterated derivative or pharmaceutically acceptable salt thereof according to any one of claims 1 to 5, which **characterized in that**, the compound can be administered together with at least one selected from glucocorticoids and mineralocorticoids.

13. The compound represented by general formula (I), or the stereoisomer, tautomer, deuterated derivative or pharmaceutically acceptable salt thereof according to any one of claims 1 to 5, which **characterized in that**, the compound can be administered together with one or more other anticancer drugs, the anticancer drugs are selected from at least one of a nonsteroidal androgen receptor antagonist, a steroid synthesis inhibitor, a chemotherapeutic agent, an estrogen receptor antagonist.
